# EUROPEAN PATENT SPECIFICATION

(11) **EP 1 636 239 B1**
(45) Date of publication and mention of the grant of the patent: **18.07.2007**
(21) Application number: 04741649.0
(22) Date of filing: 26.05.2004
(51) Int. Cl.: C07D 498/04

(54) **INDOLE DERIVATIVES WITH AN IMPROVED ANTIPSYCHOTIC ACTIVITY**
INDOLDERIVATE MIT VERBESSERTER ANTIPSYCHOTISCHER WIRKUNG
DERIVES D'INDOLE AVEC ACTIVITE ANTIPSYCHOTIQUE AMELIOREE

(30) Priority: 30.05.2003 WO PCT/EP03/05789
(43) Date of publication of application: 22.03.2006
(73) Proprietor: JANSSEN PHARMACEUTICA N.V., 2340 Beerse (BE)
(72) Inventor: BARTOLOMÉ-NEBREDA, José, M. Johnson&Johnson Pharm., 45007 Toledo (ES); ANDRÉS-GIL, José, Ignacio Johnson&Johnson Pharm., 45007 Toledo (ES)
(74) Representative: Van Borm, Werner August H.M.
(86) International application number: PCT/EP2004/050922
(87) International publication number: WO 2004/106346

(56) References cited:
- WO-A-00/40580
- WO-A-00/40581
- WO-A-96/24596
- WO-A-02/085896
- WO-A-02/085911
- GB-A- 1 570 374
- TAYLOR E W ET AL: "MOLECULAR DETERMINANTS FOR RECOGNITION OF RU 24969 ANALOGS AT CENTRAL 5-HYDROXYTRYPTAMINE RECOGNITION SITES: USE OF A BILINEAR FUNCTION AND SUBSTITUENT VOLUMES TO DESCRIBE STERIC FIT" MOLECULAR PHARMACOLOGY, BALTIMORE, MD, US, vol. 34, no. 1, 1 July 1988 (1988-07-01), pages 42-53, XP000572816 ISSN: 0026-895X

## Description

### Field of the Invention

The present invention relates to novel indol derivatives with a binding affinity towards dopamine receptors, in particular towards dopamine D₂, D₃ and D₄ receptors, with selective serotonin reuptake inhibition (SSRI) properties and acting as 5-HT_{1A} agonists or partial agonists, pharmaceutical compositions comprising the compounds according to the invention, the use thereof for the prevention and/or treatment of a range of psychiatric and neurological disorders, in particular certain psychotic disorders, most in particular schizophrenia and processes for their production.

### Background of the Invention

It is generally accepted that dopamine receptors, in particular dopamine D₂, D₃ and D₄ receptors control a large number of pharmacological events in the human body. For example, altered functions of these receptors not only participate in the genesis of psychosis, but also of anxiety, emesis, motoric functions, addiction, sleep, feeding, learning, memory, sexual behaviour, regulation of immunological responses and blood pressure.

All currently available antipsychotics have central D₂ antagonism in common. Central D₂ antagonism is therefore considered as a prerequisite for antipsychotic activity.

It has been proposed that extrapyramidal side effects could be reduced by drugs that (also) interact with the dopamine D₃ and D₄ receptors.

Dopamine D₃ receptors belong to the family of dopamine D₂-like receptors. Dopamine D₃ antagonistic properties of an antipsychotic drug could reduce the negative symptoms and cognitive deficits and result in an improved side effect profile with respect to extrapyramidal side effects and hormonal changes.

Dopamine D₄ receptors also belong to the family of dopamine D₂-like receptors which are considered to be responsible for the antipsychotic effects of a neuroleptic. Dopamine D₄ receptors are primarily located in areas of the brain other than *striatum,* suggesting that dopamine D₄ receptor ligands have antipsychotic effects and are devoid of extrapyramidal side effects.

Furthermore, in common practice the majority of schizophrenic patients is treated both with antipsychotics (central D₂-antagonists) as well as with antidepressants, predominantly selective serotonin (5-HT) reuptake inhibitors (SSRIs) (see e.g. EP 830 864 A1 by Eli Lilly). SSRIs are a well-known class of antidepressants and useful for the treatment of panic disorders and social phobia.

Furthermore, the compounds of the present invention have also been found to exhibit 5-HT_{1A} binding affinity. Clinical and pharmacological studies have shown that 5-HT_{1A} agonists and partial agonists are useful in the treatment of a range of affective disorders such as generalised anxiety disorder, panic disorder, obsessive compulsive disorder, depression and aggression.

The effect of a combined administration of a compound exhibiting SSRI behaviour and acting as a 5-HT_{1A} receptor antagonist has also been evaluated in several studies which have indicated that compounds having a combined 5-HT_{1A} receptor antagonist and SSRI activity would produce a more rapid onset of therapeutic action.

Accordingly, agents acting simultaneously as dopamine D₂, D₃ and/or D₄ antagonists, as SSRIs and as 5-HT_{1A}-agonists, partial agonists or antagonists may be particularly useful for the treatment of various psychiatric and neurological disorders, in particular certain psychotic disorders, most in particular schizophrenia with improved antipsychotic activity.

### Background prior art

WO 99/55672 (American Home Products Corporation) discloses antipsychotic indole derivatives having D₂-receptor and 5-HT_{1A} receptor affinity. The herein disclosed compounds differ from the compounds according to the present invention in the substitution of the piperazinyl-moiety.

WO 03/002552 (Lundbeck A/S) and WO 03/002556 (Lundbeck A/S) disclose antipsychotic indole derivatives having dopamine D₃ and D₄-receptor and 5-HT_{1A}-receptor affinity. The herein disclosed compounds differ from the compounds according to the present invention in the substitution pattern of the piperazinyl-moiety.

Compounds having only reported SSRI and 5-HT_{1A} potency and having an indolyl- or indolyl-like moiety (such as an 1*H*-pyrrolo[2,3-b]pyridinyl-moiety) coupled to a cyclic amine moiety such as a piperazinyl-moiety or to a linear amine moiety, such as an ethylamine-moiety have been reported in WO 99/55672 (American Home Products Corporation), WO 00/40580 (American Home Products Corporation), WO 00/40581 (American Home Products Corporation), WO 00/64898 (American Home Products Corporation), EP 1 078 928 A1 (Adir et Compagnie), US 6,313,126 (American Home. Products Corporation), WO 02/085911 (Wyeth), WO 02/40465 (Wyeth), WO 02/48105 (Wyeth) and WO 03/010169 (Wyeth). None of these compounds have reported dopamine activity.

GB 1 570 374 (Laboratoires SAUBA) describes piperazino-3-indoles as an antiinflammatory, analgesic and antipyretic drug. The compounds of the invention differ in the nature of the heterocycle, attached to the -CH₂-Y- moiety and position of the attachment.

### Description of the Invention

It was the object of the present invention to provide compounds with a binding affinity towards dopamine receptors, in particular towards dopamine D₂, D₃ and D₄ receptors. Preferentially, said compounds should also exhibit selective serotonin reuptake inhibition properties and should be acting as 5-HT_{1A} agonists or partial agonists.

This goal was achieved by the present novel indol derivatives according to Formula (I) 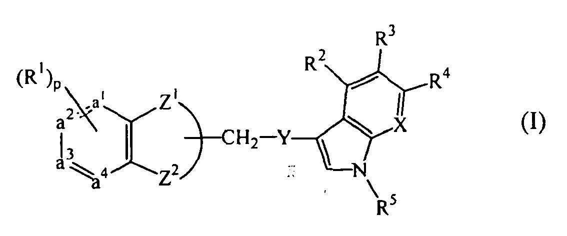 a pharmaceutically acceptable acid or base addition salt thereof, a stereochemically isomeric form thereof, an N-oxide form thereof or a quaternary ammonium salt thereof,
wherein
- -a¹=a²-a³=a⁴-: is a bivalent radical of formula

-N=CH-CH=CH- (a-1),

-CH=N-CH=CH- (a-2),

-CH=CH-N=CH- (a-3)

or

-CH=CH-CH=N- (a-4) ;
- -Z¹-Z²-: is a bivalent radical of formula

-O-CH₂-O- (b-1),

-O-CH₂-CH₂-O- (b-2),

-NR⁷-CH₂-CH₂-O- (b-3),

-O-CH₂-CH₂-NR⁷- (b-4),

-NR⁷-CH₂-CH₂-NR⁷- (b-5)

or

-S-CH2-CH₂-O- (b-6);

wherein R⁷ is selected from the group of hydrogen, hydroxy, alkyl, alkyloxyalkyl and alkylcarbonyl ;
- X: is CR⁶ or N ;
- each R¹, R², R³, R⁴ and R⁶: is independently from each other selected from the group of hydrogen, halo, cyano, nitro, alkyl, alkenyl, mono- or dialkylaminoalkyl, hydroxy, alkyloxy, alkylcarbonyloxy, amino, mono- or dialkylamino, formylamino, alkylcarbonylamino, alkylsulfonylamino, hydroxycarbonyl, alkyloxycarbonyl, aminocarbonyl, mono- or dialkylaminocarbonyl, alkylcarbonyloxy alkyloxycarbonyloxy, alkylthio, aryl and heteroaryl ;
- p: is an integer equal to 0, 1, 2 or 3 ;
- R⁵: is hydrogen or alkyl ;
- Y: is a bivalent radical of formula 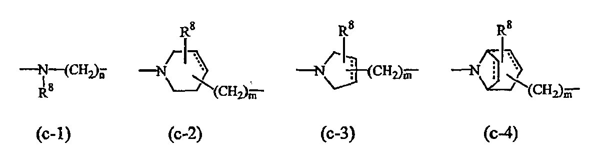 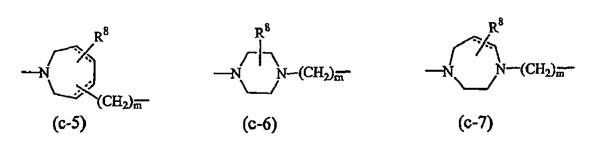 wherein
m is an integer equal to 0 or 1 ;
n is an integer equal to 0, 1, 2, 3, 4, 5 or 6 ;
the dotted line represents an optional double bond;
R⁸ is selected from the group of hydrogen, halo, alkyl, hydroxy, alkyloxy, alkylcarbonyloxy, alkyloxycarbonyloxy, hydroxycarbonyl, aminocarbonyl, mono- or dialkylaminocarbonyl, alkyloxycarbonyl and amino;
alkyl represents a straight or branched saturated hydrocarbon radical having from 1 to 6 carbon atoms or a cyclic saturated hydrocarbon radical having from 3 to 6 carbon atoms ; said radical being optionally substituted with one or more phenyl, halo, cyano, oxo, hydroxy, formyl or amino radicals ;
alkenyl represents a straight or branched unsaturated hydrocarbon radical having from 1 to 6 carbon atoms or a cyclic unsaturated hydrocarbon radical having from 3 to 6 carbon atoms ; said radical having one or more double bonds and said radical being optionally substituted with one or more phenyl, halo, cyano, oxo, hydroxy, formyl or amino radicals ;
aryl represents phenyl or naphthyl, optionally substituted with one or more radicals selected from the group of alkyl, halo, cyano, oxo, hydroxy, alkyloxy and amino ; and
heteroaryl represents a monocyclic heterocyclic radical selected from the group of azetidinyl, pyrrolidinyl, dioxolyl, imidazolidinyl, pyrrazolidinyl, piperidinyl, homopiperidinyl, dioxyl, morpholinyl, dithianyl, thiomorpholinyl, piperazinyl, imidazolidinyl, tetrahydrofuranyl, 2H-pyrrolyl, pyrrolinyl, imidazolinyl, pyrrazolinyl, pyrrolyl, imidazolyl, pyrazolyl, triazolyl, furanyl, thienyl, oxazolyl, isoxazolyl, thiazolyl, thiadiazolyl, isothiazolyl, gyridinyl, pyrimidinyl, pyrazinyl, pyridazinyl and triazinyl ; each radical optionally substituted with one or more radicals selected from the group of alkyl, aryl, arylalkyl, halo, cyano, oxo, hydroxy, alkyloxy and amino.

Compounds wherein simultaneously -a¹=a²-a³=a⁴- is (a-4), -Z¹-Z²- is (b-2) and Y is (c-2) are excluded from the scope of the present application by way of a disclaimer. Said compounds are disclosed in WO 02/085911 (Wyeth).

The invention also relates to a pharmaceutical composition comprising a pharmaceutically acceptable carrier or diluent and, as active ingredient, a therapeutically effective amount of a compound according to the invention.

The invention also relates to the use of a compound according to the invention for the preparation of a medicament for the prevention and/or treatment of a disorder or disease responsive to the inhibition of dopamine D₂, D₃ and/or D₄-receptors.

The invention also relates to the use of a compound according to the invention for the preparation of a medicament for the prevention and/or treatment of a disorder or disease responsive to the inhibition of serotonin reuptake and antagonism of 5-HT_{1A} receptors.

The invention also relates to the use of a compound according to the invention for the preparation of a medicament for the prevention and/or treatment of a disorder or disease responsive to the combined effect of a dopamine D₂, D₃ and/or D₄ antagonist, an SSRI and a 5-HT_{1A}-agonists, partial agonist or antagonist.

In particular, the invention relates to the use of a compound according to the invention for the preparation of a medicament for the prevention and/or treatment of affective disorders such as general anxiety disorder, panic disorder, obsessive compulsive disorder, depression, social phobia and eating disorders ; and other psychiatric disorders such as, but not limited to psychosis and neurological disorders.

More in particular, the invention relates to the use of a compound according to the invention for the preparation of a medicament for the prevention and/or treatment of schizophrenia.

### Detailed description of the invention

In a preferred embodiment, the invention relates to a compound according to general Formula (I), a pharmaceutically acceptable acid or base addition salt thereof, a stereochemically isomeric form thereof, an N-oxide form thereof or a quaternary ammonium salt thereof, wherein -a¹=a²-a³=a⁴- is a bivalent radical of formula (a-3) or (a-4).

In a further preferred embodiment, the invention relates to a compound according to general Formula (I), a pharmaceutically acceptable acid or base addition salt thereof, a stereochemically isomeric form thereof, an N-oxide form thereof or a quaternary ammonium salt thereof, wherein -Z¹-Z²- is a bivalent radical of formula (b-1), (b-2) or (b-3) wherein R⁷ is hydrogen or methyl.

In a further preferred embodiment, the invention relates to a compound according to general Formula (I), a pharmaceutically acceptable acid or base addition salt thereof, a stereochemically isomeric form thereof, an N-oxide form thereof or a quaternary ammonium salt thereof, wherein Y is a bivalent radical of formula (c-1) wherein n = 3 or (c-2) wherein m = 0 and R⁸ is hydrogen.

In a further preferred embodiment, the invention relates to a compound according to general Formula (I), a pharmaceutically acceptable acid or base addition salt thereof, a stereochemically isomeric form thereof, an N-oxide form thereof or a quaternary ammonium salt thereof, wherein X is CR⁶; R², R³, R⁴ and R⁶ are each independently hydrogen, halo, cyano, nitro or hydroxy and R⁵ is hydrogen.

In a further preferred embodiment, the invention relates to a compound according to general Formula (I), a pharmaceutically acceptable acid or base addition salt thereof, a stereochemically isomeric form thereof, an N-oxide form thereof or a quaternary ammonium salt thereof, wherein -a¹=a²-a³=a⁴- is a bivalent radical of formula (a-3) or (a-4); -Z¹-Z²- is a bivalent radical of formula (b-1), (b-2) or (b-3) wherein R⁷ is hydrogen or methyl ; Y is a bivalent radical of formula (c-1) wherein n = 3 or (c-2) wherein m = 0 and R⁸ is hydrogen; X is CR⁶; R², R³, R⁴ and R⁶ are each independently hydrogen, halo, cyano, nitro or hydroxy and R⁵ is hydrogen.

In the framework of this application, alkyl is defined as a monovalent straight or branched saturated hydrocarbon radical having from 1 to 6 carbon atoms, for example methyl, ethyl, propyl, butyl, 1-methylpropyl, 1,1-dimethylethyl, pentyl, hexyl ; alkyl further defines a monovalent cyclic saturated hydrocarbon radical having from 3 to 6 carbon atoms, for example cyclopropyl, methylcyclopropyl, cyclobutyl, cyclopentyl and cyclohexyl. The definition of alkyl also comprises an alkyl radical that is optionally substituted on one or more carbon atoms with one or more phenyl, halo, cyano, oxo, hydroxy, formyl and amino radicals, for example hydroxyalkyl, in particular hydroxymethyl and hydroxyethyl and polyhaloalkyl, in particular difluoromethyl and trifluoromethyl.

In the framework of this application, alkenyl is defined as an alkyl radical as defined above further comprising one or more double bonds, for example ethenyl, propenyl, butenyl, pentenyl, hexenyl, cyclopropenyl, methylcyclopropenyl, cyclobutenyl, cyclopentenyl and cyclohexenyl. The definition of alkenyl also comprises an alkenyl radical that is optionally substituted on one or more carbon atoms with one or more phenyl, halo, cyano, oxo, hydroxy, formyl and amino radicals, for example hydroxyalkenyl, in particular hydroxyethenyl.

In the framework of this application, halo is generic to fluoro, chloro, bromo and iodo.

In the framework of this application, with "compounds according to the invention" is meant a compound according to the general Formula (I), a pharmaceutically acceptable acid or base addition salt thereof, a stereochemically isomeric form thereof, an *N*-oxide form thereof or a quaternary ammonium salt thereof.

The pharmaceutically acceptable acid addition salts are defined to comprise the therapeutically active non-toxic acid addition salts forms that the compounds according to Formula (I) are able to form. Said salts can be obtained by treating the base form of the compounds according to Formula (I) with appropriate acids, for example inorganic acids, for example hydrohalic acid, in particular hydrochloric acid, hydrobromic acid, sulphuric acid, nitric acid and phosphoric acid; organic acids, for example acetic acid, hydroxyacetic acid, propanoic acid, lactic acid, pyruvic acid, oxalic acid, malonic acid, succinic acid, maleic acid, fumaric acid, malic acid, tartaric acid, citric acid, methanesulfonic acid, ethanesulfonic acid, benzenesulfonic acid, p-toluenesulfonic acid, cyclamic acid, salicylic acid, p-aminosalicylic acid and pamoic acid.

Conversely said acid addition salt forms can be converted into the free base form by treatment with an appropriate base.

The compounds according to Formula (I) containing acidic protons may also be converted into their therapeutically active non-toxic metal or amine addition salts forms (base addition salts) by treatment with appropriate organic and inorganic bases. Appropriate base salts forms comprise, for example, the ammonium salts, the alkaline and earth alkaline metal salts, in particular lithium, sodium, potassium, magnesium and calcium salts, salts with organic bases, e.g. the benzathine, *N*-methyl-D-glucamine, hybramine salts, and salts with amino acids, for example arginine and lysine.

Conversely, said salts forms can be converted into the free forms by treatment with an appropriate acid.

Quaternary ammonium salts of compounds according to Formula (I) defines said compounds which are able to form by a reaction between a basic nitrogen of a compound according to Formula (I) and an appropriate quaternizing agent, such as, for example, an optionally substituted alkylhalide, arylhalide or arylalkylhalide, in particular methyliodide and benzyliodide. Other reactants with good leaving groups may also be used, such as, for example, alkyl trifluoromethanesulfonates, alkyl methanesulfonates and alkyl p-toluenesulfonates. A quaternary ammonium salt has a positively charged nitrogen. Pharmaceutically acceptable counterions include chloro, bromo, iodo, trifluoroacetate and acetate ions.

The term addition salt as used in the framework of this application also comprises the solvates that the compounds according to Formula (I) as well as the salts thereof, are able to form. Such solvates are, for example, hydrates and alcoholates.

The *N*-oxide forms of the compounds according to Formula (I) are meant to comprise those compounds of Formula (I) wherein one or several nitrogen atoms are oxidized to the so-called N-oxide, particularly those N-oxides wherein one or more tertiary nitrogens (e.g. of the piperazinyl or piperidinyl radical) are *N*-oxidized. Such *N*-oxides can easily be obtained by a skilled person without any inventive skills and they are obvious alternatives for the compounds according to Formula (I) since these compounds are metabolites, which are formed by oxidation in the human body upon uptake. As is generally known, oxidation is normally the first step involved in drug metabolism (Textbook of Organic Medicinal and Pharmaceutical Chemistry, 1977, pages 70- 75). As is also generally known, the metabolite form of a compound can also be administered to a human instead of the compound per se, with much the same effects.

The compounds of Formula (I) may be converted to the corresponding *N*-oxide forms following art-known procedures for converting a trivalent nitrogen into its *N*-oxide form. Said N-oxidation reaction may generally be carried out by reacting the starting material of Formula (I) with an appropriate organic or inorganic peroxide. Appropriate inorganic peroxides comprise, for example, hydrogen peroxide, alkali metal or earth alkaline metal peroxides, e.g. sodium peroxide, potassium peroxide; appropriate organic peroxides may comprise peroxy acids such as, for example, benzenecarboperoxoic acid or halo substituted benzenecarboperoxoic acid, e.g. 3-chlorobenzenecarboperoxoic acid, peroxoalkanoic acids, e.g. peroxoacetic acid, alkylhydroperoxides, e.g. *tert*-butyl hydroperoxide. Suitable solvents are, for example, water, lower alkanols, e.g. ethanol and the like, hydrocarbons, e.g. toluene, ketones, e.g. 2-butanone, halogenated hydrocarbons, e.g. dichloromethane, and mixtures of such solvents.

The term "stereochemically isomeric forms" as used hereinbefore defines all the possible isomeric forms that the compounds of Formula (I) may possess. Unless otherwise mentioned or indicated, the chemical designation of compounds denotes the mixture of all possible stereochemically isomeric forms, said mixtures containing all diastereomers and enantiomers of the basic molecular structure. More in particular, stereogenic centers may have the R- or S-configuration; substituents on bivalent cyclic (partially) saturated radicals may have either the cis- or trans-configuration. Compounds encompassing double bonds can have an E or Z-stereochemistry at said double bond. Stereochemically isomeric forms of the compounds of Formula (I) are obviously intended to be embraced within the scope of this invention.

Following CAS nomenclature conventions, when two stereogenic centers of known absolute configuration are present in a molecule, an R or S descriptor is assigned (based on Cahn-Ingold-Prelog sequence rule) to the lowest-numbered chiral center, the reference center. The configuration of the second stereogenic center is indicated using relative descriptors [*R*,R*]* or [*R*,S*],* where R* is always specified as the reference center and [*R*,R**] indicates centers with the same chirality and [*R*,S**] indicates centers of unlike chirality. For example, if the lowest-numbered chiral center in the molecule has an S configuration and the second center is *R,* the stereo descriptor would be specified as *S-*[*R**,*S**]. If "α" and "β" are used : the position of the highest priority substituent on the asymmetric carbon atom in the ring system having the lowest ring number, is arbitrarily always in the "α" position of the mean plane determined by the ring system. The position of the highest priority substituent on the other asymmetric carbon atom in the ring system (hydrogen atom in compounds according to Formula (I)) relative to the position of the highest priority substituent on the reference atom is denominated "α", if it is on the same side of the mean plane determined by the ring system, or "β", if it is on the other side of the mean plane determined by the ring system.

The invention also comprises derivative compounds (usually called "pro-drugs") of the pharmacologically-active compounds according to the invention, which are degraded *in vivo* to yield the compounds according to the invention. Pro-drugs are usually (but not always) of lower potency at the target receptor than the compounds to which they are degraded. Pro-drugs are particularly useful when the desired compound has chemical or physical properties that make its administration difficult or inefficient. For example, the desired compound may be only poorly soluble, it may be poorly transported across the mucosal epithelium, or it may have an undesirably short plasma half-life. Further discussion on pro-drugs may be found in Stella, V. J. et al., "Prodrugs", Drug Delivery Systems, 1985, pp. 112-176, and Drugs, 1985, 29, pp. 455-473.

Pro-drugs forms of the pharmacologically-active compounds according to the invention will generally be compounds according to Formula (I), the pharmaceutically acceptable acid or base addition salts thereof, the stereochemically isomeric forms thereof and the *N*-oxide form thereof, having an acid group which is esterified or amidated. Included in such esterified acid groups are groups of the formula -COOR^{x}, where R^{x} is a C₁₋₆alkyl, phenyl, benzyl or one of the following groups :

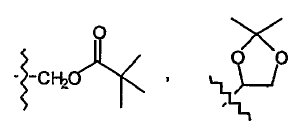

Amidated groups include groups of the formula - CONR^{y}R^{z}, wherein R^{y} is H, C₁₋₆alkyl, phenyl or benzyl and R^{z} is -OH, H, C₁₋₆alkyl, phenyl or benzyl. Compounds according to the invention having an amino group may be derivatised with a ketone or an aldehyde such as formaldehyde to form a Mannich base. This base will hydrolyze with first order kinetics in aqueous solution.

### Preparation

The compounds according to the invention can generally be prepared by a succession of steps, each of which is known to the skilled person.

The compounds of the present invention can generally be prepared by alkylating an intermediate of Formula (III) with an intermediate of Formula (II), wherein W is an appropriate leaving group such as, for example, halo, e.g. fluoro, chloro, bromo, iodo, or in some instances W may also be a sulfonyloxy group, e.g. methanesulfonyloxy, benzenesulfonyloxy, trifluoromethanesulfonyloxy and the like reactive leaving groups. The reaction can be performed in a reaction-inert solvent such as, for example, acetonitrile or tetrahydrofuran, and optionally in the presence of a suitable base such as, for example, sodium carbonate, potassium carbonate, calciumoxide or triethylamine. Stirring may enhance the rate of the reaction. The reaction may conveniently be carried out at a temperature ranging between room temperature and the reflux temperature of the reaction mixture and, if desired, the reaction may be carried out in an autoclave at an increased pressure.

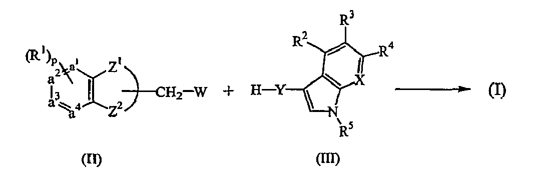

Compounds of Formula (I) can also be prepared by reductively aminating an intermediate of Formula (IV) following art-known reductive amination procedures with an intermediate of Formula (III).

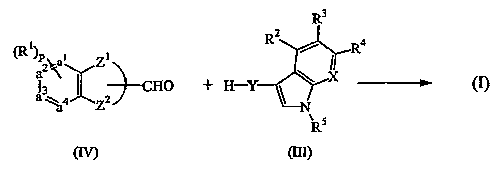

Said reductive amination can be performed in a reaction-inert solvent such as, for example, dichloromethane, ethanol, toluene or a mixture thereof, and in the presence of a reducing agent such as, for example, a borohydride, e.g. sodium borohydride, sodium cyanoborohydride or triacetoxy borohydride. It may also be convenient to use hydrogen as a reducing agent in combination with a suitable catalyst such as, for example, palladium-on-charcoal, rhodium-on-carbon or platinum-on-charcoal. In case hydrogen is used as reducing agent, it may be advantageous to add a dehydrating agent to the reaction mixture such as, for example, aluminium *tert*-butoxide. In order to prevent the undesired further hydrogenation of certain functional groups in the reactants and the reaction products, it may also be advantageous to add an appropriate catalyst-poison to the reaction mixture, e.g., thiophene or quinoline-sulphur. To enhance the rate of the reaction, the temperature may be elevated in a range between room temperature and the reflux temperature of the reaction mixture and optionally the pressure of the hydrogen gas may be raised.

Alternatively, compounds of Formula (I) can also be prepared by reacting an acid chloride of Formula (V) with an intermediate of Formula (III) under suitable reaction conditions, followed by reduction of the corresponding amide intermediate formed following art-known reduction procedures.

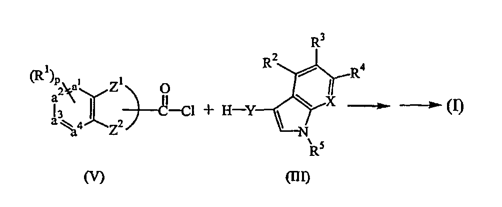

Said acylation reaction can be performed in a reaction-inert solvent such as, for example, acetonitrile, dichloromethane, dimethylformamide, or tetrahydrofuran, or a mixture thereof, and in the presence of a suitable base such as, for example, pyridine, diisopropylethylamine or triethylamine. Stirring may enhance the rate of the reaction. The reaction may conveniently be carried out at a temperature ranging between room temperature and the reflux temperature of the reaction mixture. Said amide reduction can be performed, under inert atmosphere conditions, in a reaction-inert solvent such as, for example, tetrahydrofuran, toluene, or diethylether, or a mixture thereof, and in the presence of a reducing agent such as, for example, a borane, e.g. boranetetrahydrofuran complex, or borane-methylsulfide complex, or an hydride, e.g. lithium aluminium hydride, diisobutyl aluminium hydride, or aluminium hydride. Stirring may enhance the rate of the reaction. The reaction may conveniently be carried out at a temperature ranging between -78°C and the reflux temperature of the reaction mixture.

The compounds of Formula (I) may further be prepared by converting compounds of Formula (I) into each other according to art-known group transformation reactions, and further, if desired, by converting the compounds of Formula (I), into a therapeutically active non-toxic acid addition salt by treatment with an acid, or into a therapeutically active non-toxic base addition salt by treatment with a base, or conversely, by converting the acid addition salt form into the free base by treatment with alkali, or converting the base addition salt into the free acid by treatment with acid; and, if desired, by preparing stereochemically isomeric forms, N-oxides thereof and quaternary ammonium salts thereof..

The starting materials and some of the intermediates are known compounds and are commercially available or may be prepared according to conventional reaction procedures generally known in the art, such as the prior art cited above or for instance, EP 714 894-A1 and J. Med. Chem. 1993, 36(9), 1194-1202.

Compounds of Formula (I) and some of the intermediates may have one or more stereogenic centers in their structure, present in a R or a S configuration, such as, e.g. the carbon atom linked to the -CH₂-Y-moiety.

The compounds of Formula (I) as prepared in the processes described below may be synthesized in the form of racemic mixtures of enantiomers that can be separated from one another following art-known resolution procedures. The racemic compounds of Formula (I) may be converted into the corresponding diastereomeric salt forms by reaction with a suitable chiral acid Said diastereomeric salt forms are subsequently separated, for example, by selective or fractional crystallization and the enantiomers are liberated there from by alkali. An alternative manner of separating the enantiomeric forms of the compounds of Formula (I) involves liquid chromatography using a chiral stationary phase. Said pure stereochemically isomeric forms may also be derived from the corresponding pure stereochemically isomeric forms of the appropriate starting materials, provided that the reaction occurs stereospecifically. Preferably if a specific stereoisomer is desired, said compound would be synthesized by stereospecific methods of preparation. These methods will advantageously employ enantiomerically pure starting materials.

### Pharmacology

The compounds according to the invention, in particular compounds according to Formula (I), the pharmaceutically acceptable acid or base addition salts thereof, a stereochemically isomeric form thereof, an N-oxide form thereof or a quaternary ammonium salt thereof, have surprisingly been shown to have a binding affinity towards dopamine receptors, in particular towards dopamine D₂, D₃ and D₄ receptors, with selective serotonin reuptake inhibition properties and acting as 5-HT_{1A} agonists or partial agonists and show a strong antidepressant and/or anxiolytic activity and/or antipsychotic activity.

*In vitro* receptor and neurotransmitter transporter binding and signal-transduction studies can be used to evaluate the dopamine antagonism activity and serotonin (5-HT) reuptake inhibitor activity of the present compounds. As indices for central penetration and potency to block the dopamine and serotonin transporters, respectively, *ex vivo* dopamine and serotonin transporter occupancy can be used. As indices of serotonin (5-HT) reuptake inhibition activity, the inhibition of head-twitches and excitation in rats, observed after subcutaneous injection or oral dosage of the compound before subcutaneous p-chloroamphetamine administration in rats can be used (pCA-test).

In view of their above mentioned potency, the compounds according to the invention are suitable for the prevention and/or treatment in diseases where either one of the activities alone or the combination of said activities may be of therapeutic use. In particular, the compounds according to the invention may be suitable for treatment and/or prophylaxis in the following diseases :
- Central nervous system disorders, including :
   - Mood disorders, including particularly major depressive disorder, depression with or without psychotic features, catatonic features, melancholic features, atypical features of postpartum onset and, in the case of recurrent episodes, with or without seasonal pattern, dysthymic disorder, bipolar I disorder, bipolar II disorder, cyclothymic disorder, recurrent brief depressive disorder, mixed affective disorder, bipolar disorder not otherwise specified, mood disorder due to a general medical condition, substance-induced mood disorder, mood disorder not otherwise specified, seasonal affective disorder and premenstrual dysphoric disorders.
   - Anxiety disorders, including panic attack, agoraphobia, panic disorder without agoraphobia, agoraphobia without history of panic disorder, specific phobia, social phobia, obsessive-compulsive disorder, posttraumatic stress disorder, acute stress disorder, generalized anxiety disorder, anxiety disorder due to a general medical condition, substance-induced anxiety disorder and anxiety disorder not otherwise specified.
   - Stress-related disorders associated with depression and/or anxiety, including acute stress reaction, adjustment disorders (brief depressive reaction, prolonged depressive reaction, mixed anxiety and depressive reaction, adjustment disorder with predominant disturbance of other emotions, adjustment disorder with predominant disturbance of conduct, adjustment disorder with mixed disturbance of emotions and conduct, adjustment disorders with other specified predominant symptoms) and other reactions to severe stress.
   - Dementia, amnesic disorders and cognitive disorders not otherwise specified, especially dementia caused by degenerative disorders, lesions, trauma, infections, vascular disorders, toxins, anoxia, vitamin deficiency or endocrinic disorders, or amnesic disorders caused by alcohol or other causes of thiamine deficiency, bilateral temporal lobe damage due to Herpes simplex encephalitis and other limbic encephalitis, neuronal loss secondary to anoxia / hypoglycaemia / severe convulsions and surgery, degenerative disorders, vascular disorders or pathology around ventricle III.
   - Cognitive disorders due to cognitive impairment resulting from other medical conditions.
   - Personality disorders, including paranoid personality disorder, schizoid personality disorder, schizotypical personality disorder, antisocial personality disorder, borderline personality disorder, histrionic personality disorder, narcissistic personality disorder, avoidant personality disorder, dependent personality disorder, obsessive-compulsive personality disorder and personality disorder not otherwise specified.
   - Schizoaffective disorders resulting from various causes, including schizoaffective disorders of the manic type, of the depressive type, of mixed type, paranoid, disorganized, catatonic, undifferentiated and residual schizophrenia, schizophreniform disorder, schizoaffective disorder, delusional disorder, brief psychotic disorder, shared psychotic disorder, substance-induced psychotic disorder and psychotic disorder not otherwise specified
   - Akinesia, akinetic-rigid syndromes, dyskinesia and medication-induced parkinsonism, Gilles de la Tourette syndrome and its symptoms, tremor, chorea, myoclonus, tics and dystonia.
   - Attention-deficit / hyperactivity disorder (ADHD).
   - Parkinson's disease, drug-induced Parkinsonism, post-encephalitic Parkinsonism, progressive supranuclear palsy, multiple system atrophy, corticobasal degeneration, parkinsonism-ALS dementia complex and basal ganglia calcification.
   - Dementia of the Alzheimer's type, with early or late onset, with depressed mood.
   - Behavioural disturbances and conduct disorders in dementia and the mentally retarded, including restlessness and agitation.
   - Extra-pyramidal movement disorders.
   - Down's syndrome.
   - Akathisia.
   - Eating Disorders, including anorexia nervosa, atypical anorexia nervosa, bulimia nervosa, atypical bulimia nervosa, overeating associated with other psychological disturbances, vomiting associated with other psychological disturbances and non-specified eating disorders.
   - AIDS-associated dementia.
   - Chronic pain conditions, including neuropathic pain, inflammatory pain, cancer pain and post-operative pain following surgery, including dental surgery. These indications might also include acute pain, skeletal muscle pain, low back pain, upper extremity pain, fibromyalgia and myofascial pain syndromes, orofascial pain, abdominal pain, phantom pain, tic douloureux and atypical face pain, nerve root damage and arachnoiditis, geriatric pain, central pain and inflammatory pain.
   - Neurodegenerative diseases, including Alzheimer's disease, Huntington's chorea, Creutzfeld-Jacob disease, Pick's disease, demyelinating disorders, such as multiple sclerosis and ALS, other neuropathies and neuralgia, multiple sclerosis, amyotropical lateral sclerosis, stroke and head trauma.
   - Addiction disorders, including:
   - Substance dependence or abuse with or without physiological dependence, particularly where the substance is alcohol, amphetamines, amphetamine-like substances, caffeine, cannabis, cocaine, hallucinogens, inhalants, nicotine, opioids, phencyclidine, phencyclidine-like compounds, sedative-hypnotics, benzodiazepines and/or other substances, particularly useful for treating withdrawal from the above substances and alcohol withdrawal delirium.
   - Mood disorders induced particularly by alcohol, amphetamines, caffeine, cannabis, cocaine, hallucinogens, inhalants, nicotine, opioids, phencyclidine, sedatives, hypnotics, anxiolitics and other substances.
   - Anxiety disorders induced particularly by alcohol, amphetamines, caffeine, cannabis, cocaine, hallucinogens, inhalants, nicotine, opioids, phencyclidine, sedatives, hypnotics, anxiolitics and other substances and adjustment disorders with anxiety.
   - Smoking cessation.
   - Body weight control, including obesity.
   - Sleep disorders and disturbances, including :
   - Dyssomnias and/or parasomnias as primary sleep disorders, sleep disorders related to another mental disorder, sleep disorder due to a general medical condition and substance-induced sleep disorder.
   - Circadian rhythms disorders.
   - Improving the quality of sleep.
   - Sexual dysfunction, including sexual desire disorders, sexual arousal disorders, orgasmic disorders, sexual pain disorders, sexual dysfunction due to a general medical condition, substance-induced sexual dysfunction and sexual dysfunction not otherwise specified.

The invention therefore relates to a compound according to the general Formula (I), a pharmaceutically acceptable acid or base addition salt thereof, a stereochemically isomeric form thereof, an N-oxide form thereof or a quaternary ammonium salt thereof, for use as a medicine.

The invention also relates to the use of a compound according to the invention for the preparation of a medicament for the prevention and/or treatment of a disorder or disease responsive to the inhibition of dopamine D₂, D₃ and/or D₄-receptors.

The invention also relates to the use of a compound according to the invention for the preparation of a medicament for the prevention and/or treatment of a disorder or disease responsive to the inhibition of serotonin reuptake and antagonism of 5-HT_{1A} receptors.

The invention also relates to the use use of a compound according to the invention for the preparation of a medicament for the prevention and/or treatment of a disorder or disease responsive to the combined effect of a dopamine D₂, D₃ and/or D₄ antagonist, an SSRI and a 5-HT_{1A}-agonists, partial agonist or antagonist.

The present invention also relates to a method for the prevention and/or treatment of dopamine-mediated diseases, in particular for the prevention and/or treatment of affective disorders such as general anxiety disorder, panic disorder, obsessive compulsive disorder, depression, social phobia and eating disorders ; and other psychiatric disorders such as, but not limited to psychosis and neurological disorders, comprising administering to a human in need of such administration an effective amount of a compound according to the invention, in particular according to Formula (I), a pharmaceutically acceptable acid or base addition salt thereof, a stereochemically isomeric form thereof, an N-oxide form thereof or a quaternary ammonium salt thereof.

More in particular, the present invention relates to the use of a compound according to the invention for the preparation of a medicament for the prevention and/or treatment of schizophrenia.

### Pharmaceutical compositions

The invention also relates to a pharmaceutical composition comprising a pharmaceutically acceptable carrier or diluent and, as active ingredient, a therapeutically effective amount of a compound according to the invention, in particular a compound according to Formula (I), a pharmaceutically acceptable acid or base addition salt thereof, a stereochemically isomeric form thereof, an N-oxide form thereof or a quaternary ammonium salt thereof

The compounds according to the invention, in particular the compounds according to Formula (I), the pharmaceutically acceptable acid or base addition salt thereof, a stereochemically isomeric form thereof, an N-oxide form thereof or a quaternary ammonium salt thereof, or any subgroup or combination thereof may be formulated into various pharmaceutical forms for administration purposes. As appropriate compositions there may be cited all compositions usually employed for systemically administering drugs. To prepare the pharmaceutical compositions of this invention, an effective amount of the particular compound, optionally in addition salt form, as the active ingredient is combined in intimate admixture with a pharmaceutically acceptable carrier, which carrier may take a wide variety of forms depending on the form of preparation desired for administration. These pharmaceutical compositions are desirable in unitary dosage form suitable, in particular, for administration orally, rectally, percutaneously, by parenteral injection or by inhalation. For example, in preparing the compositions in oral dosage form, any of the usual pharmaceutical media may be employed such as, for example, water, glycols, oils, alcohols and the like in the case of oral liquid preparations such as suspensions, syrups, elixirs, emulsions and solutions; or solid carriers such as starches, sugars, kaolin, diluents, lubricants, binders, disintegrating agents and the like in the case of powders, pills, capsules and tablets. Because of their ease in administration, tablets and capsules represent the most advantageous oral dosage unit forms in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. Also included are solid form preparations that are intended to be converted, shortly before use, to liquid form preparations. In the compositions suitable for percutaneous administration, the carrier optionally comprises a penetration enhancing agent and/or a suitable wetting agent, optionally combined with suitable additives of any nature in minor proportions, which additives do not introduce a significant deleterious effect on the skin. Said additives may facilitate the administration to the skin and/or may be helpful for preparing the desired compositions. These compositions may be administered in various ways, e.g., as a transdermal patch, as a spot-on, as an ointment.

It is especially advantageous to formulate the aforementioned pharmaceutical compositions in unit dosage form for ease of administration and uniformity of dosage. Unit dosage form as used herein refers to physically discrete units suitable as unitary dosages, each unit containing a predetermined quantity of active ingredient calculated to produce the desired therapeutic effect in association with the required pharmaceutical carrier. Examples of such unit dosage forms are tablets (including scored or coated tablets), capsules, pills, powder packets, wafers, suppositories, injectable solutions or suspensions and the like, and segregated multiples thereof.

Since the compounds according to the invention are potent orally administrable dopamine antagonists, pharmaceutical compositions comprising said compounds for administration orally are especially advantageous.

The following examples are intended to illustrate but not to limit the scope of the present invention.

### Experimental part

Hereinafter "RT" means room temperature, . "DME" is defined as 1,2-dimethoxyethane, "DIPE" is defined as diisopropyl ether, "THF" is defined as tetrahydrofurane and "LDA" is defined as Lithiumdiisopropylamide.

### A. Preparation of the intermediate compounds

### Example A.1

### a. Preparation of intermediate compound 1

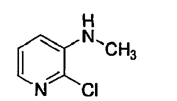

To a solution of 2-chloro-3-pyridinamine (0.0465 mol) in THF (45 ml) at -78 °C under N₂ flow, LDA 2.0M (0.0513 mol) was added dropwise. The mixture was allowed to warm to 0 °C and was stirred for 1 hour and then cooled to -78 °C. Then CH₃I (0.0582 mol) was added and the reaction mixture was allowed to warm to room temperature and was stirred for 16 hours. A saturated NH₄Cl-solution was added and the mixture was extracted with AcOEt. The separated organic layer was dried (Na₂SO₄), filtered and the solvent was evaporated. The residue was purified by short open column chromatography over silica gel (eluent: hexane/AcOEt 80/20). The product fractions were collected and the solvent was evaporated. Yield : 5.91 g of intermediate compound 1 (89%).

### b. Preparation of intermediate compound 2

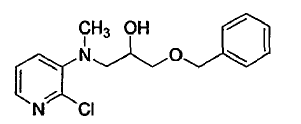

To a solution of intermediate compound 1 (0.031 mol) in THF (50 ml) under N₂ flow at -78 °C, C (0.062 mol) was slowly added. The reaction mixture was allowed to warm to 0 °C and was stirred for 1 hour. After cooling again to -78 °C, a solution of [(phenylmethoxy)methyl]-oxirane (0.034 mol) in THF (40 ml) was added and the mixture was allowed to warm to room temperature and was stirred for 16 hours. A saturated NH₄Cl-solution was added and the mixture was extracted with AcOEt. The separated organic layer was dried (Na₂SO₄), filtered and the solvent was evaporated. The residue was purified by short open column chromatography over silica gel (eluent : hexane/AcOEt 50/50). The product fractions were collected and the solvent was evaporated. Yield : 7.18 g of intermediate compound 2 (75 %).

### c. Preparation of intermediate compound 3

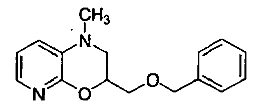

To a suspension of NaH 60 % (0.081 mol) in DME (250 ml), a solution of intermediate compound 2 (0.023 mol) in DME (250 ml) was added dropwise. The reaction mixture was stirred and refluxed for 16 hours. After cooling, the mixture was taken up in H₂O/AcOEt. The separated organic layer was dried (Na₂SO₄), filtered and the solvent was evaporated. The residue was purified by short open column chromatography over silica gel (eluent : hexane/AcOEt 95/5). The desired fractions were collected and the solvent was evaporated. Yield : 5.82 g of intermediate compound 3 (93 %).

### d. Preparation of intermediate compound 4

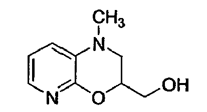

A mixture of intermediate compound 3 (0.018 mol) and FeCl₃ (0.036 mol) in CH₂Cl₂ (500 ml) was stirred at room temperature for 16 hours. Then FeCl₃ (0.018 mol) was added and the mixture was stirred for 6 hours more. Extra FeCl₃ (0.018 mol) was added again and the mixture was stirred for 16 hours. The reaction mixture was basified with a saturated NH₄OH-solution and the formed precipitate was filtered over dicalite. The separated organic layer was extracted with a saturated NH₄Cl-solution, dried (Na₂SO₄), filtered and the solvent was evaporated. The residue was purified by short open column chromatography over silica gel (eluent : CH₂Cl₂/(MeOH/NH₃) 95/5). The product fractions were collected and the solvent was evaporated. The residue was purified again by short open column chromatography over silica gel (eluent : AcOEt/(MeOH/NH₃) 98/2;95/5). The desired fractions were collected and the solvent was evaporated. Yield : 2.1 g of intermediate compound 4 (65 %).

### e. Preparation of intermediate compound 5

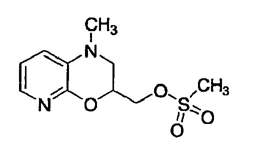

To a solution of intermediate compound 4 (0.0111 mol) and Et₃N (0.0222 mol) in CH₂Cl₂ (200 ml) at 0 °C, methanesulfonylchloride (0.0166 mol) was added dropwise. The reaction mixture was stirred at 0 °C for 1 hour. Then H₂O was added. The separated organic layer was extracted with brine, dried (Na₂SO₄), filtered and the solvent was evaporated. Yield : 2.85 g intermediate compound 5.

### Example A.2

### a. Preparation of intermediate compound 6

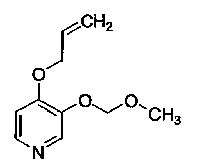

Under nitrogen atmosphere. 2-propen-1-ol (0.002 mol) was added dropwise to a stirred mixture of NaH, 6 0% (0.002 mol) in DME (5 ml). The mixture was stirred at room temperature for 15 minutes. A solution of 4-chloro-3-(methoxymethoxy)-pyridine (0.0017 mol) in DME (5 ml) was added dropwise. The resulting reaction mixture was stirred at reflux overnight. The mixture was washed with water and extracted with EtOAc. The organic layer was dried (Na₂SO₄), filtered and evaporated till dryness. The residue was purified by open column chromatography (eluent: hexane/EtOAc 3/2; CH₂Cl₂/2-propanone 90/10; CH₂Cl₂/MeOH 96/4). The product fractions were collected and the solvent was evaporated. Yield: 0.18 g of intermediate compound 6 (54 %).

### b. Preparation of intermediate compound 7

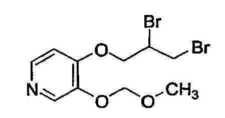

Br₂ (1.32 ml; 0.0258 mol) was added dropwise to a solution of intermediate compound 6 (5.04 g; 0.0258 mol) in CH₂Cl₂ (56 ml). The reaction mixture was stirred at room temperature for 15 minutes. The mixture was poured into a saturated NaHCO₃ solution with a few drops of a 10 % Na₂SO₄ solution. This mixture was extracted. The organic layer was dried over Na₂SO₄, filtered and evaporated till dryness. Yield: 8.3 g of intermediate compound 7 (90 %).

### c. Preparation of intermediate compound 8

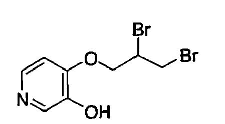

A mixture of intermediate compound 7 (0.0248 mol), HCl, 3N (35.42 ml)and EtOH (40 ml) was stirred at room temperature overnight The reaction mixture was concentrated under vacuum. The concentrate was cooled on an ice-water bath. The mixture was neutralized with a saturated NaHCO₃ solution and extracted with EtOAc. The organic layer was dried (Na₂SO₄), filtered and evaporated till dryness. The residue was purified by open column chromatography (eluent: CH₂Cl₂; CH₂Cl₂/MeOH (98/2, 96/4 and 90/10)). The pure fractions were collected and the solvent was evaporated. Yield: 4.27 g of intermediate compound 8 (55 %)

### d. Preparation of intermediate compound 9

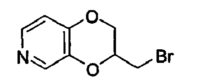

A solution of intermediate compound 8 (0.0097 mol) in EtOH (50ml) was stirred and refluxed overnight. NaHCO₃ (0.0097 mol) was added and the resulting reaction mixture was stirred and refluxed overnight. The solvent was evaporated. The residue was washed with water and extracted with CH₂Cl₂. The organic layer was dried (Na₂SO₄), filtered and the solvent was evaporated. The residue was purified by open column chromatography (eluent: hexane/EtOAc (3/2)). The pure fractions were collected and the solvent was evaporated. Yield: 1.51 g of intermediate compound 9 (67 %).

### B. Preparation of the final compounds

### Example B.1

### Preparation of final compound 1

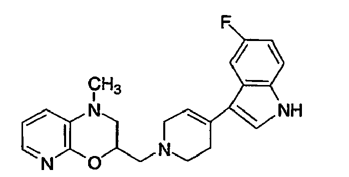

A solution of intermediate compound 5 (prepared according to A1.e) (0.00193 mol) and 5-fluoro-3-(1,2,3,6-tetrahydro-4-pyridinyl)-1*H*-indole (0.0038 mol) in dioxane (10 ml) and ethanol (10 ml) was stirred for 3 days at 100 °C. The solvent was evaporated. The residue was washed with water, then extracted with CH₂Cl₂. The separated organic layer was dried (Na₂SO₄), filtered and the solvent evaporated. The residue was purified by flash column chromatography over silica gel (eluent: CH₂Cl₂/CH₃OH 99.5/0.5, 99/1, 98/2 and 97/3). The product fractions were collected and the solvent was evaporated. Yield: 0.27 g of final compound 1 (37 %).

### Example B.2

### Preparation of final compound 5

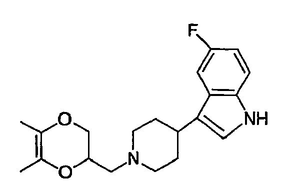

A mixture of intermediate compound 9 (prepared according to A2.d) (0.0014 mol), 5-fluoro-3-(4-piperidinyl)-9-1*H*-indole (0.0016 mol) and *N*-ethyl-*N*-(1-methylethyl)-2-propanamine (0.0028 mol) in ethanol (3 ml) and 1,4-dioxane (3 ml) was stirred for 4 days at 100 °C in a sealed tube. The solvent was evaporated. The residue was taken up into CH₂Cl₂, then extracted with brine. The separated organic layer was dried (Na₂SO₄), filtered and the solvent evaporated. The residue was purified using a Sep-Pak fitted with 10 g of silica gel in a manifold under vacuum (eluent: CH₂Cl₂/(CH₃OH/NH₃) 99/1 and 97.5/2.5). The product fractions were collected and the solvent was evaporated. The residue was dissolved in ethanol and converted into the ethanedioic acid salt (2:3). The precipitate was filtered off, washed with DIPE, and dried. Yield: 0.126 g of final compound 5 (20 %).

Table 1 lists the compounds of Formula (I) which were prepared according to one of the above described examples. Compound I is a comparative example from WO 02/085911 (Wyeth).

**Table I**

| | | | | | | |
|---|---|---|---|---|---|---|
| 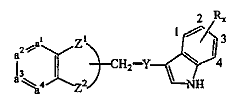 | | | | | | |

| Co. No. | Ex. No. | a¹=a²-a³=a⁴ | -Z¹-Z²- | Y | Rₓ | Physical data |
|---|---|---|---|---|---|---|
| 1 | B1 | CH=CH-CH=N | 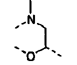 | 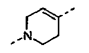 | 2-F | 250°C (decomp.) |
| 2 | B1 | CH=CH.CH=N | 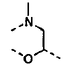 | 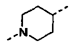 | 2-F | Oxalate Decomp. |
| 3 | B1 | CH=CH-CH=N | 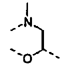 | 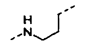 | 2-F | Oxalate 191.3°C Decomp |
| 12 | B2 | CH=CH-N=CH | 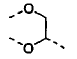 | 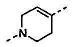 | 1-F | Oxalate foam |
| 4 | B2 | CH=CH-N=CH | 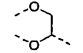 | 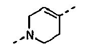 | 2-F | 198.8°C |
| 13 | B2 | CH=CH-N=CH | 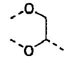 | 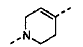 | 2-Br | Oxalate foam |
| 14 | B2 | CH=CH-N=CH | 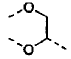 | 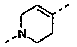 | 2-NO₂ | Racemic mixture Oxalate Foam |
| 15 | B2 | CH=CH-N=CH | 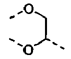 | 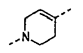 | 2-NO₂ | A enantiomer Decomposition [a]_{D}²⁰=-25.4° (C=0.70; DMF) |
| 16 | B2 | CH=CH-N=CH | 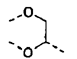 | 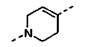 | 2-NO₂ | B enantiomer 214.6°C [n]_{D}²⁰=+28.9° (C=0.71; DMF) |
| 17 | B2 | CH=CH-N=CH | 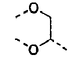 | 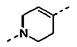 | 4-F | Oxalate foam |
| 5 | B2 | CH-CH-N-CH | 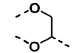 | 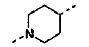 | 2-F | Oxalate 202.4°C |
| 18 | B2 | CH=CH-N=CH | 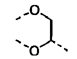 | 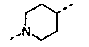 | 2-Cl | Oxalate foam |
| 19 | B2 | CH=CH-N=CH | 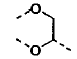 | 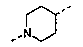 | 2-OH | 110.7°C |
| 20 | B2 | CH=CH-N=CH | 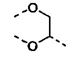 | 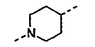 | 2-CN | Foam Racemic mixture |
| 21 | B2 | CH=CH-N=CH | 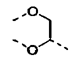 | 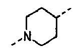 | 2-CN | A enantiomer Foam [a]_{D}²⁰ = 23.0° (C=0.68; DMF) |
| 22 | B2 | CH=CH-N=CH | 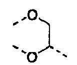 | 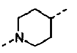 | 2-CN | B enantiomer Decomposition [a]_{D}²⁰=+22.7° (C=0.72; DMF) |
| 23 | B2 | CH=CH-N=CH | 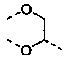 | 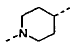 | 3-F | Oxalate foam |
| 24 | B2 | CH=CH-N=CH | 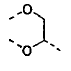 | 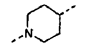 | 4-F | Foam |
| 6 | B2 | CH=CH-N=CH | 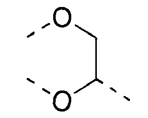 | 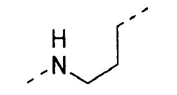 | 2-F | Oxalate 203.6°C |
| 25 | B2 | CH=CH-N=CH | 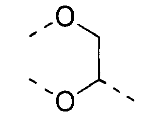 | 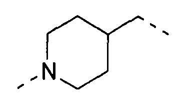 | 2-F | Oxalate foam |
| 7 | B1 | CH=CH-CH=N | 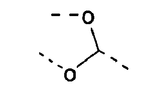 | 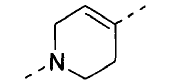 | 2-F | Decomp. |
| 8 | B1 | CH=CH-CH=N | 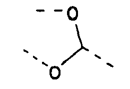 | 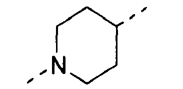 | 2-F | |
| 9 | B1 | CH=CH-CH=N | 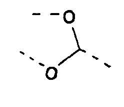 | 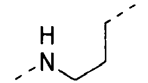 | 2-F | Oxalate Foam |
| 11 | B1 | CH=CH-CH=N | 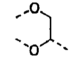 | 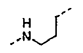 | 2-F | Oxalate Decomp. |

| Comparative Example | | | | | | |
|---|---|---|---|---|---|---|
| I | B1 | CH=CH-CH=N | 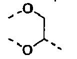 | 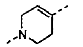 | 2-F | 208.5°C |

### C. Pharmacological example

### General

The interaction of the compounds of Formula (I) with dopamine-receptors, h5HT-transporter and h5HT_{1A} receptor was assessed in *in vitro* radioligand binding experiments. In general, a low concentration of a radioligand with a high binding affinity for a particular receptor or transporter is incubated with a sample of a tissue preparation enriched in a particular receptor or transporter or with a preparation of cells expressing cloned human receptors in a buffered medium. During the incubation, the radioligand binds to the receptor or transporter. When equilibrium ofbinding is reached, the receptor bound radioactivity is separated from the non-bound radioactivity, and the receptor- or transporter-bound activity is counted. The interaction of the test compounds with the receptor is assessed in competition binding experiments. Various concentrations of the test compound are added to the incubation mixture containing the receptor- or transporter preparation and the radioligand. The test compound in proportion to its binding affinity and its concentration inhibits binding of the radioligand.

### Example c.1 : Binding experiment for dopamine D₂, D₃ and D₄ receptors

### hD₂

Human Dopamine D_{2L} receptor-transfected CHO cells were collected by scraping into ice-cold Tris-HCl buffer (50 mM, pH 7.4). The suspension was centrifuged (23 500 x g, 10 min, 4 °C) and pellets stored at -70 °C until required. They were then thawed and briefly homogenised using an Ultra-Turrax T25 homogeniser prior to dilution to an appropriate protein concentration optimised for specific and non-specific binding. [³H]Spiperone (NEN, specific activity ∼70 Ci/mmol) was diluted in Tris-HCl assay buffer containing NaCl, CaCl₂, MgCl₂, KCl (50, 120, 2, 1, and 5 mM respectively, adjusted to pH 7.7 with HCl) at a concentration of 2 nmol/L. Prepared radioligand (50 µl) was then incubated (30 min, 37 °C) with membrane preparations pre-diluted to an appropriate protein concentration (400 µl), and with 50 µl of either the 10 % DMSO control, Butaclamol (10⁻⁶ mol/L final concentration), or compound of interest. Membrane-bound activity was detected by filtration through a Packard Filtermate harvester onto Unifilterplates, washing with ice-cold Tris-HCl buffer (50 mM; pH8.0; 3 x 4 ml). Filters were allowed to dry before adding scintillation fluid and counting in a Topcount scintillation counter. % Specific bound and competition binding curves were calculated using S-Plus software (Insightful).

### hD₃

Human Dopamine D₃ receptor-transfected CHO cells were collected by scraping into ice-cold Tris-HCl buffer (50 mM, pH 7.4). The suspension was centrifuged (23 500 x g, 10 min, 4 °C) and pellets stored at -70 °C until required. They were then thawed and briefly homogenised using an Ultra-Turrax T25 homogeniser prior to dilution to an appropriate protein concentration optimised for specific and non-specific binding. [¹²⁵I]Iodosulpride (Amersham, specific activity 2000 Ci/mmol) was diluted in Tris-HCl assay buffer containing NaCl, CaCl₂, MgCl₂, KCl and BSA (50, 120, 2, 1, 5 mM, 0.1 % respectively, adjusted to pH 7.7 with HCI) at a concentration of 2 nmol/L. Prepared radioligand (20 µl) was incubated (60 min, RT) and with 20 µl of either the 10 % DMSO control, Risperidone (10⁻⁶ mol/L final concentration), or compound of interest, then with membrane preparations (80 µl). Overnight incubation followed after addition of WGA-coated PVT SPA beads (250 µl; Amersham) and membrane-bound counts measured in a Wallac Microbeta. % Specific bound and competition binding curves were calculated using S-Plus software (Insightful).

### hD₄

Human Dopamine D₄ receptor-transfected Sf9 cells were collected by scraping into ice-cold Tris-HCl buffer (50 mM, pH 7.4). The suspension was centrifuged (23 500 x g, 10 min, 4 °C) and pellets stored at-70 °C until required. They were then thawed and briefly homogenised using an Ultra-Turrax T25 homogeniser prior to dilution to an appropriate protein concentration optimised for specific and non-specific binding. [³H]Spiperone (Amersham; specific activity -70 Ci/mmol) was diluted in Tris-HCl assay buffer containing NaCl, CaCl₂, MgCl₂, KCl (50, 120, 2, 1, and 5 mM respectively; pH 7.4) at a concentration of 5 nmol/L. Prepared radioligand (50 µl) was then incubated (30 min, 37 °C) with membrane preparations (400 µl) and with 50 µl of either the 10 % DMSO control, Haloperidol (10⁻⁵ mol/L final concentration), or compound of interest. Membrane-bound activity was collected by filtration through a Packard Filtermate harvester onto Unifilterplates, washing with ice-cold Tris-HCl buffer (50 mM; pH 7.7; 3 x 4 ml). Filters were allowed to dry before adding scintillation fluid and counting in a Topcount scintillation counter. % Specific bound and competition binding curves were calculated using S-Plus software (Insightful).

### Example c.2 : Binding experiment for 5-HT transporter

Frozen human 5HT transporter-transfected HEK cells (Perkin Elmer, Brussels) were thawed and briefly homogenised using an Ultra-Turrax T25 homogeniser prior to dilution to an appropriate protein concentration optimised for specific and non-specific binding. [³H]Paroxetine (NEN, specific activity 20 Ci/mmol) was diluted in Tris-HCl assay buffer containing NaCl and KCl (50 mM, 120 mM and 5 mM, respectively; pH 7.4) at a concentration of 5 nmol/L. Prepared radioligand (25 µl) was then incubated (60 min, 25°C) with membrane preparations (200 µl) and with 25 µl of either 10 % DMSO control, Imipramine (10⁻⁶mol/L final concentration), or compound of interest Membrane-bound activity was detected by filtration through a Packard Filtermate harvester onto Unifilterplates pre-soaked in 0.1 % PEI, washing with ice-cold assay buffer (3 x 4 ml). Filters were dried prior to addition of scintillation fluid and then counting in a Topcount scintillation counter. % Specific bound and competition binding curves were calculated using S-Plus software (Insightful).

### Example c.2 : Binding experiment for 5-HT_{1A} receptor

Human 5HT_{1A} receptor-transfected L929 cells were collected by scraping into ice-cold Tris-HCl buffer (50 mM, pH 7.4). The suspension was centrifuged (23 500 x g, 10 min, 4 °C) and pellets stored at-70 °C until required. They were then thawed and briefly homogenised using an Ultra-Turrax T25 homogeniser prior to dilution to an appropriate protein concentration optimised for specific and non-specific binding. [³H]8OHDPAT (NEN, specific activity 127 Ci/mmol) was diluted in Tris-HCl assay buffer containing CaCl₂ (50 mM and 4 mM, respectively; pH 7.7) at a concentration of 5 nmol/L. Prepared radioligand (50 µl) was then incubated (30 min, 37 °C) with membrane preparations from L929 cells stably-transfected with the h5HT1A gene construct (400 µl) and with 50 µl of either the 10 % DMSO control, spiroxatrine (10⁻⁶ mol/L final concentration), or compound of interest. Membrane-bound activity was detected by filtration through a Packard Filtermate harvester onto Unifilterplates, washing with ice-cold Tris-HCl buffer (3 × 4 ml), followed by drying. Scintillation fluid was added and membranes were counted in a Topcount scintillation counter. % Specific bound and competition binding curves were calculated using S-Plus software (Insightful).

The data for the compounds tested have been summarized in Table 2.

From Table 2 can be seen that the compounds of the invention exhibit a higher dopamine D₂ and/or D₃ and/or D₄ antagonist activity combined with a higher SSRI and/or 5HT_{1A} activity compared to the compounds of the prior art.

**Table 2. Pharmacological data for the compounds according to the invention.**

| **Co. No.** | **pIC₅₀** | | | | |
|---|---|---|---|---|---|
| | **D₂** | **D₃** | **D₄** | **5HT reuptake inhibition** | **5HT_{1A}** |
| **1** | 6.7 | 7.1 | - | 7.8 | 7.9 |
| **2** | 6.4 | 7.0 | - | 6.5 | 7.8 |
| **3** | < 6 | 6.4 | - | 6.7 | 8.5 |
| **12** | - | 6.8 | | 7.0 | - |
| **4** | 6.8 | - | 7.3 | 7.3 | 6.9 |
| **13** | 6.8 | - | - | 7.0 | - |
| **14** | 7.0 | - | - | 7.0 | 6.7 |
| **15** | 6.7 | - | - | 8.0 | - |
| **16** | 6.5 | - | - | 6.6 | - |
| **17** | 6.9 | - | - | 7.3 | - |
| **5** | 7.1 | 8.2 | - | 6.9 | 7.4 |
| **18** | 7.5 | - | - | 6.7 | - |
| **19** | 7.2 | - | - | 6.5 | - |
| **20** | 7.2 | - | - | 7.4 | - |
| **21** | 7.1 | - | - | 7.7 | 7.5 |
| **22** | 6.1 | - | - | 6.3 | 8.0 |
| **23** | 7.6 | - | - | 6.6 | - |
| **24** | 7.6 | - | - | 6.5 | - |
| **6** | 6.7 | 7.5 | - | 6.5 | 8.2 |
| **25** | 6.1 | - | - | 7.2 | - |
| **7** | 6.6 | 7.8 | 6.3 | < 6 | 6.4 |
| **9** | < 6 | 6.6 | - | 6.3 | 7.5 |
| **11** | 6.8 | 6.4 | - | 6.5 | 8.9 |

| **Comparative Example** | | | | | |
|---|---|---|---|---|---|
| **I** | 6.6 | 6.3 | - | 6.7 | 6.9 |

### D. Composition examples

"Active ingredient" (a.i.) as used throughout these examples relates to a compound of Formula (I), the pharmaceutically acceptable acid or base addition salts thereof, the stereochemically isomeric forms thereof, the *N*-oxide form thereof and prodrugs thereof.

### Example D.1 : Oral drops

500 Grams of the a.i. is dissolved in 0.5 l of 2-hydroxypropanoic acid and 1.5 l of the polyethylene glycol at 60-80 °C. After cooling to 30-40 °C there are added 35 l of polyethylene glycol and the mixture is stirred well. Then there is added a solution of 1750 grams of sodium saccharin in 2.5 l of purified water and while stirring there are added 2.5 l of cocoa flavor and polyethylene glycol q.s. To a volume of 50 l, providing an oral drop solution comprising 10 mg/ml of a.i. the resulting solution is filled into suitable containers.

### Example D.2: oral solution

9 Grams of methyl 4-hydroxybenzoate and 1 gram of propyl 4-hydroxybenzoate are dissolved in 4 l of boiling purified water. In 3 l of this solution are dissolved first 10 grams of 2,3-dihydroxybutanedioic acid and thereafter 20 grams of the a.i. the latter solution is combined with the remaining part of the former solution and 12 11,2,3-propanetriol and 3 l of sorbitol 70% solution are added thereto. 40 Grams of sodium saccharin are dissolved in 0.5 l of water and 2 ml of raspberry and 2 ml of gooseberry essence are added. The latter solution is combined with the former, water is added q.s. to a volume of 201 providing an oral solution comprising 5 mg of the active ingredient per teaspoonful (5 ml). The resulting solution is filled in suitable containers.

### Example D.3 : film-coated tablets

### Preparation of tablet core

A mixture of 100 grams of the a.i., 570 grams lactose and 200 grams starch is mixed well and thereafter humidified with a solution of 5 grams sodium dodecyl sulfate and 10 grams polyvinylpyrrolidone in about 200 ml of water. The wet powder mixture is sieved, dried and sieved again. Then there is added 100 grams microcrystalline cellulose and 15 grams hydrogenated vegetable oiL The whole is mixed well and compressed into tablets, giving 10.000 tablets, each containing 10 mg of the active ingredient.

### Coating

To a solution of 10 grams methyl cellulose in 75 ml of denaturated ethanol there is added a solution of 5 grams of ethyl cellulose in 150 ml of dichloromethane. Then there are added 75 ml of dichloromethane and 2.5 ml 1,2,3-propanetriol. 10 grams of polyethylene glycol is molten and dissolved in 75 ml of dichloromethane. The latter solution is added to the former and then there are added 2.5 grams of magnesium octadecanoate, 5 grams of polyvinylpyrrolidone and 30 ml of concentrated color suspension and the whole is homogenated. The tablet cores are coated with the thus obtained mixture in a coating apparatus.

### Example D.4 : Injectable solution

1.8 Grams methyl 4-hydroxybenzoate and 0.2 grams propyl 4-hydroxybenzoate are dissolved in about 0.5 l of boiling water for injection. After cooling to about 50 °C there are added while stirring 4 grams lactic acid, 0.05 grams propylene glycol and 4 grams of the ai.. The solution is cooled to room temperature and supplemented with water for injection q.s. ad 1 l, giving a solution comprising 4 mg/ml of a.i.. The solution is sterilized by filtration and filled in sterile containers.

## Claims

1. Indol derivatives according to Formula (I) 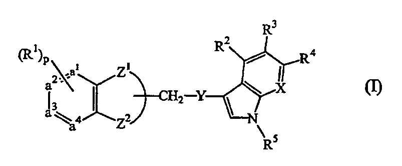 a pharmaceutically acceptable acid or base addition salt thereof, a stereochemically isomeric form thereof, an N-oxide form thereof or a quaternary ammonium salt thereof, wherein
-a¹=a²-a³=a⁴- is a bivalent radical of formula
-N=CH-CH=CH- (a-1),
-CH=N-CH=H- (a-2),
-CH=CH-N=CH- (a-3)
or
-CH=CH-CH=N- (a-4);
-Z¹-Z²- is a bivalent radical of formula
-O-CH₂-O- (b-1),
-O-CH₂-CH₂-O- (b-2),
-NR⁷-CH₂-CH₂-O- (b-3),
-O-CH₂-CH₂-NR⁷- (b-4),
-NR⁷-CH₂-CH₂-NR⁷- (b-5)
or
-S-CH2-CH₂-O- (b-6);
wherein R⁷ is selected from the group of hydrogen, hydroxy, alkyl, alkyloxyalkyl and alkylcarbonyl ;
X is CR⁶ or N ;
each R¹, R², R³, R⁴ and R⁶ is independently from each other selected from the group of hydrogen, halo, cyano, nitro, alkyl, alkenyl, mono- or dialkylaminoalkyl, hydroxy, alkyloxy, alkylcarbonyloxy, amino, mono- or dialkylamino, formylamino, alkylcarbonylamino, alkylsulfonylamino, hydroxycarbonyl, alkyloxycarbonyl, aminocarbonyl, mono- or dialkylaminocarbonyl, alkylcarbonyloxy alkyloxycarbonyloxy, alkylthio, aryl and heteroaryl ;
p is an integer equal to 0, 1, 2 or 3 ;
R⁵ is hydrogen or alkyl ;
Y is a bivalent radical of formula 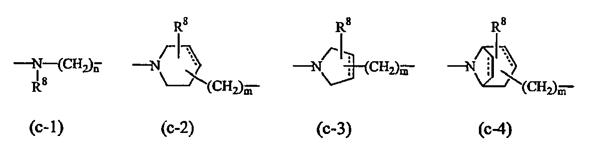 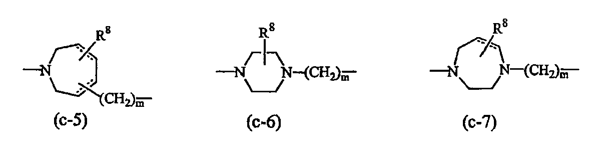 wherein
m is an integer equal to 0 or 1 ;
n is an integer equal to 0, 1, 2, 3, 4, 5 or 6 ;
the dotted line represents an optional double bond;
R⁸ is selected from the group of hydrogen, halo, alkyl, hydroxy, alkyloxy, alkylcarbonyloxy, alkyloxycarbonyloxy, hydroxycarbonyl, aminocarbonyl, mono- or dialkylaminocarbonyl, alkyloxycarbonyl and amino ;
alkyl represents a straight or branched saturated hydrocarbon radical having from 1 to 6 carbon atoms or a cyclic saturated hydrocarbon radical having from 3 to 6 carbon atoms ; said radical being optionally substituted with one or more phenyl, halo, cyano, oxo, hydroxy, formyl or amino radicals ;
alkenyl represents a straight or branched unsaturated hydrocarbon radical having from 1 to 6 carbon atoms or a cyclic unsaturated hydrocarbon radical having from 3 to 6 carbon atoms ; said radical having one or more double bonds and said radical being optionally substituted with one or more phenyl, halo, cyano, oxo, hydroxy, formyl or amino radicals;
aryl represents phenyl or naphthyl, optionally substituted with one or more radicals selected from the group of alkyl, halo, cyano, oxo, hydroxy, alkyloxy and amino ; and
heteroaryl represents a monocyclic heterocyclic radical selected from the group of azetidinyl, pyrrolidinyl, dioxolyl, imidazolidinyl, pyrrazolidinyl, piperidinyl, homopiperidinyl, dioxyl, morpholinyl, dithianyl, thiomorpholinyl, piperazinyl, imidazolidinyl, tetrahydrofuranyl, 2H-pyrrolyl, pyrrolinyl, imidazolinyl, pyrrazolinyl, pyrrolyl, imidazolyl, pyrazolyl, triazolyl, furanyl, thienyl, oxazolyl, isoxazolyl, thiazolyl, thiadiazolyl, isothiazolyl, pyridinyl, pyrimidinyl, pyrazinyl, pyridazinyl and triazinyl ; each radical optionally substituted with one or more radicals selected from the group of alkyl, aryl, arylalkyl, halo, cyano, oxo, hydroxy, alkyloxy and amino ;
with the proviso that compounds wherein simultaneously -a¹=a²-a³=a⁴- is (a-4), -Z¹-Z²- is (b-2) and Y is (c-2) are excluded.

2. Compound according to claim 1, **characterized in that** -a¹=a²-a³=a⁴- is a bivalent radical of formula (a-3) or (a-4).

3. Compound according to any one of claims 1 and 2, **characterized in that** -Z¹-Z²- is a bivalent radical of formula (b-1), (b-2) or (b-3) wherein R⁷ is hydrogen or methyl.

4. Compound according to any one of claims 1 to 3, **characterized in that** Y is a bivalent radical of formula (c-1) wherein n= 3 or (c-2) wherein m = 0 or 1 and R⁸ is hydrogen.

5. Compound according to any one of claims 1 to 4, **characterized in that** X is CR⁶ ; R², R³, R⁴ and R⁶ are each independently hydrogen, halo, cyano, nitro or hydroxy and R⁵ is hydrogen.

6. Compound according to any one of claims 1 to 5, **characterized in that** -a¹=a²-a³=a⁴- is a bivalent radical of formula (a-3) or (a-4) ; -Z¹-Z²- is a bivalent radical of formula (b-1), (b-2) or (b-3) wherein R⁷ is hydrogen or methyl ; Y is a bivalent radical of formula (c-1) wherein n = 3 or (c-2) wherein m = 0 or 1 and R⁸ is hydrogen; X is CR⁶ ; R², R³, R⁴ and R⁶ are each independently hydrogen, halo, cyano, nitro or hydroxy and R⁵ is hydrogen.

7. Compound according to any one of claims 1 to 6 for use as a medicine.

8. A pharmaceutical composition comprising a pharmaceutically acceptable carrier or diluent and, as active ingredient, a therapeutically effective amount of a compound according to any one of claims 1 to 6.

9. The use of a compound according to any one of claims 1 to 6 for the preparation of a medicament for the prevention and/or treatment of a disorder or disease responsive to the inhibition of dopamine D₂, D₃ and/or D₄-receptors.

10. The use of a compound according to any one of claims 1 to 6 for the preparation of a medicament for the prevention and/or treatment of a disorder or disease responsive to the inhibition of serotonin reuptake and antagonism of 5-HT_{1A} receptors.

11. The use of a compound according to any one of claims 1 to 6 for the preparation of a medicament for the prevention and/or treatment of a disorder or disease responsive to the combined effect of a dopamine D₂, D₃ and/or D₄ antagonist, an SSRI and a 5-HT_{1A}-agonists, partial agonist or antagonist.

12. The use of a compound according to any one of claims 1 to 6 for the preparation of a medicament for the prevention and/or treatment of affective disorders such as general anxiety disorder, panic disorder, obsessive compulsive disorder, depression, social phobia and eating disorders ; and other psychiatric disorders such as, but not limited to psychosis and neurological disorders.

13. The use of a compound according to any one of claims 1 to 6 for the preparation of a medicament for the prevention and/or treatment of schizophrenia.

14. Process for the preparation of a compound according to Formula (I) **characterized by** either
(a) alkylating an intermediate of Formula (III) with an intermediate of Formula (II), wherein all variables are defined as in claim 1 and W is an appropriate leaving group, in a reaction-inert solvent and optionally in the presence of a suitable base ; 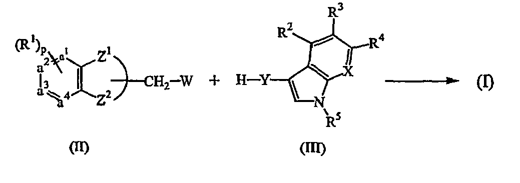
(b) reductively aminating an intermediate of Formula (IV) is with an intermediate of Formula (III) in a reaction-inert solvent and in the presence of a reducing agent 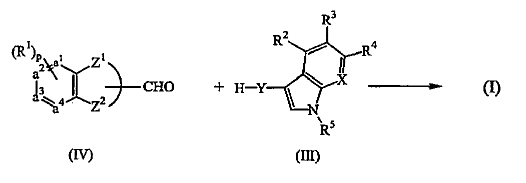
(c) reacting an acid chloride of Formula (V) with an intermediate of Formula (III) in a reaction-inert solvent and in the presence of a suitable base, followed by reduction of the corresponding amide intermediate formed in a reaction-inert solvent and in the presence of a reducing agent; 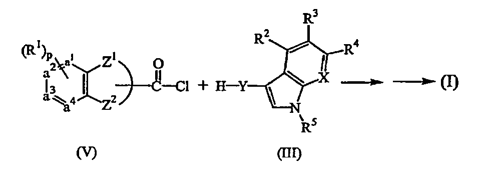
(d) and, if desired, converting compounds of Formula (I) into each other following art-known transformations, and further, if desired, converting the compounds of Formula (I), into a therapeutically active non-toxic acid addition salt by treatment with an acid, or into a therapeutically active non-toxic base addition salt by treatment with a base, or conversely, converting the acid addition salt form into the free base by treatment with alkali, or converting the base addition salt into the free acid by treatment with acid; and, if desired, preparing stereochemically isomeric forms, *N*-oxides thereof and quaternary ammonium salts thereof.

## Patentansprüche

1. Indolderivate der Formel (I) 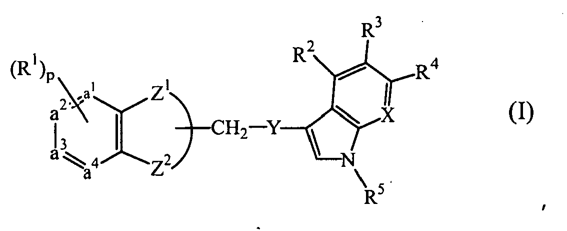 deren pharmazeutisch unbedenkliche Säure- und Basenadditionssalze, deren stereochemisch isomere Formen, deren N-Oxidformen und deren quaternäre Ammoniumsalze, wobei
-a¹=a²-a³=a⁴- für einen zweiwertigen Rest der Formel
-N=CH-CH=CH- (a-1),
-CH=N-CH=CH- (a-2),
-CH=CH-N=CH- (a-3)
oder
-CH=CH-CH=N- (a-4)
steht;
-Z¹-Z²- für einen zweiwertigen Rest der Formel
-O-CH₂-O- (b-1),
-O-CH₂-CH₂-O- (b-2),
-NR⁷-CH₂-CH₂-O- (b-3),
-O-CH₂-CH₂-NR⁷- (b-4),
-NR⁷-CH₂-CH₂-NR⁷ (b-5)
oder
-S-CH₂-CH₂-O- (b-6)
steht; wobei R⁷ aus der aus Wasserstoff, Hydroxy, Alkyl, Alkyloxyalkyl und Alkylcarbonyl bestehenden Gruppe ausgewählt ist;
X für CR⁶ oder N steht;
R¹, R², R³, R⁴ und R⁶ jeweils unabhängig voneinander aus der aus Wasserstoff, Halogen, Cyano, Nitro, Alkyl, Alkenyl, Mono- oder Dialkylaminoalkyl, Hydroxy, Alkyloxy, Alkylcarbonyloxy, Amino, Mono- oder Dialkylamino, Formylamino, Alkylcarbonylamino, Alkylsulfonylamino, Hydroxycarbonyl, Alkyloxycarbonyl, Aminocarbonyl, Mono- oder Dialkylaminocarbonyl, Alkylcarbonyloxy, Alkyloxycarbonyloxy, Alkylthio, Aryl und Heteroaryl bestehenden Gruppe ausgewählt sind;
p für eine ganze Zahl gleich 0, 1, 2 oder 3 steht;
R⁵ für Wasserstoff oder Alkyl steht;
Y für einen zweiwertigen Rest der Formel 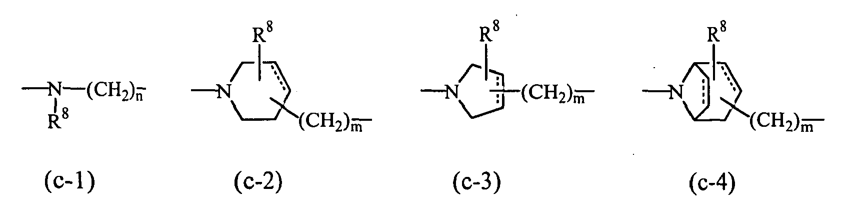 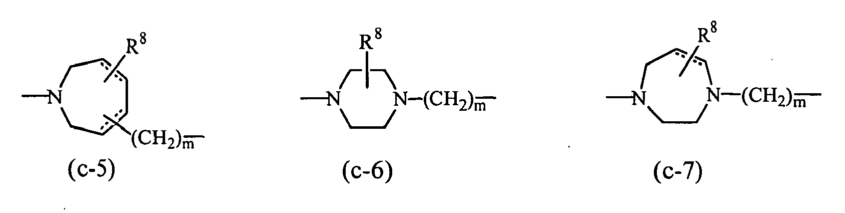 steht,
wobei
m für eine ganze Zahl gleich 0 oder 1 steht;
n für eine ganze Zahl gleich 0, 1, 2, 3, 4, 5 oder 6 steht;
die gestrichelte Linie für eine gegebenenfalls vorhandene Doppelbindung steht;
R⁸ aus der aus Wasserstoff, Halogen, Alkyl, Hydroxy, Alkyloxy, Alkylcarbonyloxy, Alkyloxycarbonyloxy, Hydroxycarbonyl, Aminocarbonyl, Mono- oder Dialkylaminocarbonyl, Alkyloxycarbonyl und Amino bestehenden Gruppe ausgewählt ist;
Alkyl für einen geradkettigen oder verzweigten gesättigten Kohlenwasserstoffrest mit 1 bis 6 Kohlenstoffatomen oder einen cyclischen gesättigten Kohlenwasserstoffrest mit 3 bis 6 Kohlenstoffatomen steht; wobei der Rest gegebenenfalls durch einen oder mehrere Phenyl-, Halogen-, Cyano-, Oxo-, Hydroxy-, Formyl- oder Aminoreste substituiert ist;
Alkenyl für einen geradkettigen oder verzweigten ungesättigten Kohlenwasserstoffrest mit 1 bis 6 Kohlenstoffatomen oder einen cyclischen ungesättigten Kohlenwasserstoffrest mit 3 bis 6 Kohlenstoffatomen steht; wobei der Rest eine oder mehrere Doppelbindungen aufweist und der Rest gegebenenfalls durch einen oder mehrere Phenyl-, Halogen-, Cyano-, Oxo-, Hydroxy-, Formyl- oder Aminoreste substituiert ist;
Aryl für Phenyl oder Naphthyl, gegebenenfalls substituiert durch einen oder mehrere Reste ausgewählt aus der aus Alkyl, Halogen, Cyano, Oxo, Hydroxy, Alkyloxy und Amino bestehenden Gruppe, steht; und
Heteroaryl für einen monocyclischen heterocyclischen Rest ausgewählt aus der aus Azetidinyl, Pyrrolidinyl, Dioxolyl, Imidazolidinyl, Pyrrazolidinyl, Piperidinyl, Homopiperidinyl, Dioxyl, Morpholinyl, Dithianyl, Thiomorpholinyl, Piperazinyl, Imidazolidinyl, Tetrahydrofuranyl, 2H-Pyrrolyl, Pyrrolinyl, Imidazolinyl, Pyrrazolinyl, Pyrrolyl, Imidazolyl, Pyrazolyl, Triazolyl, Furanyl, Thienyl, Oxazolyl, Isoxazolyl, Thiazolyl, Thiadiazolyl, Isothiazolyl, Pyridinyl, Pyrimidinyl, Pyrazinyl, Pyridazinyl und Triazinyl bestehenden Gruppe steht; wobei die Reste jeweils gegebenenfalls durch einen oder mehrere Reste ausgewählt aus der aus Alkyl, Aryl, Arylalkyl, Halogen, Cyano, Oxo, Hydroxy, Alkyloxy und Amino bestehenden Gruppe substituiert sind;
mit der Maßgabe, daß Verbindungen, in denen gleichzeitig -a¹=a²-a³=a⁴ für (a-4), -Z¹-Z²- für (b-2) und Y für (c-2) steht, ausgeschlossen sind.

2. Verbindungen nach Anspruch 1, **dadurch gekennzeichnet, daß** -a¹=a²-a³=a⁴- für einen zweiwertigen Rest der Formel (a-3) oder (a-4) steht.

3. Verbindungen nach Anspruch 1 oder 2, **dadurch gekennzeichnet, daß** -Z¹-Z²- für einen zweiwertigen Rest der Formel (b-1), (b-2) oder (b-3) steht, wobei R⁷ für Wasserstoff oder Methyl steht.

4. Verbindungen nach einem der Ansprüche 1 bis 3, **dadurch gekennzeichnet, daß** Y für einen zweiwertigen Rest der Formel (c-1), wobei n = 3, oder (c-2), wobei m = 0 oder 1 und R⁸ für Wasserstoff steht, steht.

5. Verbindungen nach einem der Ansprüche 1 bis 4, **dadurch gekennzeichnet, daß** X für CR⁶ steht; R², R³, R⁴ und R⁶ jeweils unabhängig voneinander für Wasserstoff, Halogen, Cyano, Nitro oder Hydroxy stehen und R⁵ für Wasserstoff steht.

6. Verbindungen nach einem der Ansprüche 1 bis 5, **dadurch gekennzeichnet, daß** -a¹=a²-a³=a⁴- für einen zweiwertigen Rest der Formel (a-3) oder (a-4) steht; -Z¹-Z²- für einen zweiwertigen Rest der Formel (b-1), (b-2) oder (b-3) steht, wobei R⁷ für Wasserstoff oder Methyl steht; Y für einen zweiwertigen' Rest der Formel (c-1), wobei n = 3, oder (c-2), wobei m = 0 oder 1 und R⁸ für Wasserstoff steht, steht; X für CR⁶ steht; R², R³, R⁴ und R⁶ jeweils unabhängig voneinander für Wasserstoff, Halogen, Cyano, Nitro oder Hydroxy stehen und R⁵ für Wasserstoff steht.

7. Verbindungen nach einem der Ansprüche 1 bis 6 zur Verwendung als Medizin.

8. Pharmazeutische Zusammensetzung, enthaltend einen pharmazeutisch unbedenklichen Träger oder ein pharmazeutisch unbedenkliches Verdünnungsmittel und, als Wirkstoff, eine therapeutisch wirksame Menge einer Verbindung nach einem der Ansprüche 1 bis 6.

9. Verwendung einer Verbindung nach einem der Ansprüche 1 bis 6 zur Herstellung eines Medikaments zur Prävention und/oder Behandlung einer Erkrankung oder Krankheit, die auf die Inhibierung von Dopamin-D₂-, -D₃- und/oder -D₄-Rezeptoren anspricht.

10. Verwendung einer Verbindung nach einem der Ansprüche 1 bis 6 zur Herstellung eines Medikaments zur Prävention und/oder Behandlung einer Erkrankung oder Krankheit, die auf die Inhibierung der Serotoninwiederaufnahme und eine Antagonisierung von 5-HT_{1A}-Rezeptoren anspricht.

11. Verwendung einer Verbindung nach einem der Ansprüche 1 bis 6 zur Herstellung eines Medikaments zur Prävention und/oder Behandlung einer Erkrankung oder Krankheit, die auf die kombinierte Wirkung eines Dopamin-D₂-, -D₃- und/oder -D₄-Antagonisten, eines SSRI und eines 5-HT_{1A}-Agonisten, -Partialagonisten oder -Antagonisten anspricht.

12. Verwendung einer Verbindung nach einem der Ansprüche 1 bis 6 zur Herstellung eines Medikaments zur Prävention und/oder Behandlung von affektiven Psychosen wie allgemeinen Angstneurosen, Angstanfällen, Zwangsneurosen, Depression, sozialen Phobien und Eßstörungen, und anderen psychiatrischen Erkrankungen wie beispielsweise, jedoch nicht darauf beschränkt, Psychosen und neurologischen Erkrankungen.

13. Verwendung einer Verbindung nach einem der Ansprüche 1 bis 6 zur Herstellung eines Medikaments zur Prävention und/oder Behandlung von Schizophrenie.

14. Verfahren zur Herstellung einer Verbindung der Formel (I), **dadurch gekennzeichnet, daß** man entweder
(a) ein Zwischenprodukt der Formel (III) in einem reaktionsinerten Lösungsmittel und gegebenenfalls in Gegenwart einer geeigneten Base mit einem Zwischenprodukt der Formel (II) alkyliert, wobei alle Variablen wie in Anspruch 1 definiert sind und W für eine geeignete Abgangsgruppe steht; 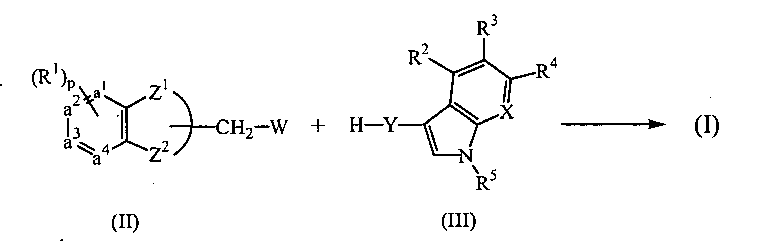
(b) ein Zwischenprodukt der Formel (IV) in einem reaktionsinerten Lösungsmittel und in Gegenwart eines Reduktionsmittels reduktiv mit einem Zwischenprodukt der Formel (III) aminiert; 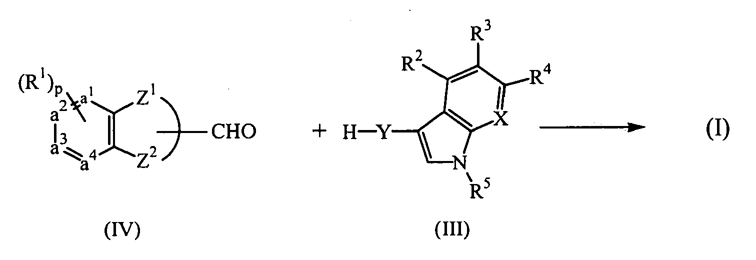
(c) ein Säurechlorid der Formel (V) in einem reaktionsinerten Lösungsmittel und in Gegenwart einer geeigneten Base mit einem Zwischenprodukt der Formel (III) umsetzt und anschließend das entsprechende gebildete Amidzwischenprodukt in einem reaktionsinerten Lösungsmittel und in Gegenwart eines Reduktionsmittels reduziert; 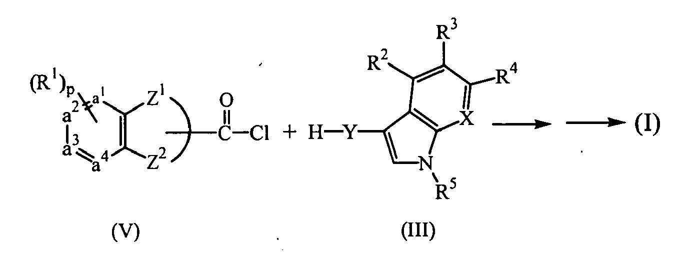
(d) und, falls gewünscht, Verbindungen der Formel (I) nach im Stand der Technik bekannten Transformationen ineinander umwandelt, und weiterhin, falls gewünscht, die Verbindungen der Formel (I) durch Behandlung mit einer Säure in ein therapeutisch wirksames, nicht toxisches Säureadditionssalz umwandelt oder durch Behandlung mit einer Base in ein therapeutisch wirksames, nicht toxisches Basenadditionssalz umwandelt, oder umgekehrt die Säureadditionssalzform durch Behandlung mit Alkali in die freie Base umwandelt oder das Basenadditionssalz durch Behandlung mit einer Säure in die freie Säure umwandelt; und, falls gewünscht, stereochemisch isomere Formen, *N-*Oxide davon und quaternäre Ammoniumsalze davon herstellt.

## Revendications

1. Dérivés d'indole selon la formule (I) 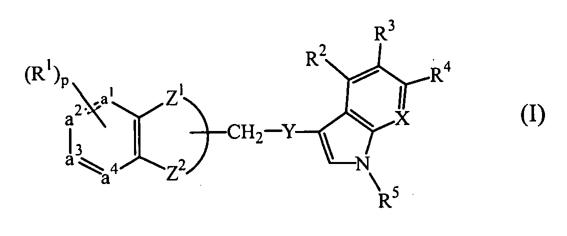 sel d'addition acide ou basique pharmaceutiquement acceptable de ces dérivés, forme isomère stéréochimique de ces dérivés, forme N-oxyde de ces dérivés ou sel d'ammonium quaternaire de ces dérivés, dans lesquels
-a¹=a²-a³=a⁴- représente un radical bivalent de formule
-N=CH-CH=CH- (a-1),
-CH=N-CH=CH- (a-2),
-CH=CH-N=CH- (a-3)
ou
-CH=CH-CH=N- (a-4) ;
-Z¹ - Z²- représente un radical bivalent de formule
-O-CH₂-O- (b-1),
-O-CH₂-CH₂-O- (b-2),
-NR⁷-CH₂-CH₂-O- (b-3),
-O-CH₂-CH₂-NR⁷- (b-4),
-NR⁷-CH₂-CH₂-NR⁷- (b-5)
ou
-S-CH₂-CH₂-O- (b-6) ;
dans laquelle R⁷ est choisi parmi le groupe constitué de l'atome d'hydrogène, des radicaux hydroxy, alkyle, alkyloxyalkyle et alkylcarbonyle ;
X représente CR⁶ ou un atome N ;
chaque R¹, R², R³, R⁴ et R⁶ sont choisis indépendamment les uns des autres parmi le groupe constitué de l'atome d'hydrogène, des radicaux halogéno, cyano, nitro, alkyle, alcényle, mono- ou dialkylaminoalkyle, hydroxy, alkyloxy, alkylcarbonyloxy, amino, mono- ou dialkylamino, formylamino, alkylcarbonylamino, alkylsulfonylamino, hydroxycarbonyle, alkyloxycarbonyle, aminocarbonyle, mono- ou dialkylaminocarbonyle, alkylcarbonyloxy, alkyloxycarbonyloxy, alkylthio, aryle et hétéroaryle ;
p vaut un nombre entier égal à 0, 1, 2 ou 3 ;
R⁵ représente un atome d'hydrogène ou un radical alkyle ;
Y représente un radical bivalent de formule 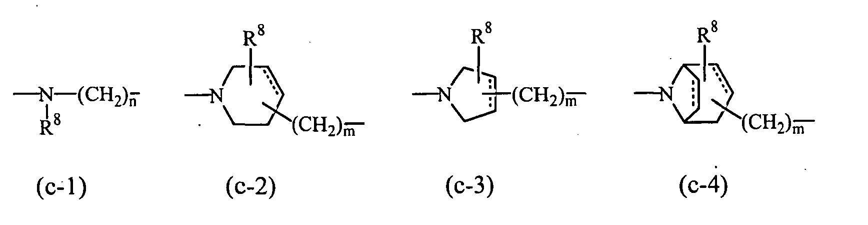 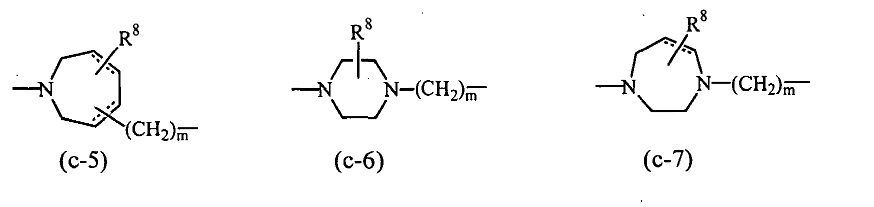 dans laquelle
m vaut un nombre entier égal à 0 ou à 1 ;
n vaut un nombre entier égal à 0, 1, 2, 3, 4, 5 ou 6 ;
la ligne pointillée représente une double liaison éventuelle ;
R⁸ est choisi parmi le groupe constitué de l'atome d'hydrogène, des radicaux halogéno, alkyle, hydroxy, alkyloxy, alkylcarbonyloxy, alkyloxycarbonyloxy, hydroxycarbonyle, aminocarbonyle, mono- ou dialkylaminocarbonyle, alkyloxycarbonyle et amino ;
alkyle représente un radical d'hydrocarbure saturé droit ou ramifié qui possède de 1 à 6 atomes de carbone ou un radical d'hydrocarbure saturé cyclique qui possède de 3 à 6 atomes de carbone ; ledit radical étant éventuellement substitué par un ou plusieurs radicaux phényle, halogéno, cyano, oxo, hydroxy, formyle ou amino ;
alcényle représente un radical d'hydrocarbure insaturé droit ou ramifié qui possède de 1 à 6 atomes de carbone ou un radical d'hydrocarbure insaturé cyclique qui possède de 3 à 6 atomes dé carbone ; ledit radical ayant une ou plusieurs doubles liaisons et ledit radical étant éventuellement substitué par un ou plusieurs radicaux phényle, halogéno, cyano, oxo, hydroxy, formyle ou amino ;
aryle représente un radical phényle ou naphtyle, éventuellement substitué par un ou plusieurs radicaux choisis parmi le groupe constitué des radicaux alkyle, halogéno, cyano, oxo, hydroxy, alkyloxy et amino ; et
hétéroaryle représente un radical monocyclique hétérocyclique choisi parmi le groupe constitué des radicaux azétidinyle, pyrrolidinyle, dioxolyle, imidazolidinyle, pyrrazolidinyle, pipéridinyle, homopipéridinyle, dioxyle, morpholinyle, dithianyle, thiomorpholinyle, pipérazinyle, imidazolidinyle, tétrahydrofuranyle, 2H-pyrrolyle, pyrrolinyle, imidazolinyle, pyrrazolinyle, pyrrolyle, imidazolyle, pyrazolyle, triazolyle, furanyle, thiényle, oxazolyle, isoxazolyle, thiazolyle, thiadiazolyle, isothiazolyle, pyridinyle, pyrimidinyle, pyrazinyle, pyridazinyle et triazinyle ; chaque radical étant éventuellement substitué par un ou plusieurs radicaux choisis parmi le groupe constitué des radicaux alkyle, aryle, arylalkyle, halogéno, cyano, oxo, hydroxy, alkyloxy et amino ;
à condition que les composés dans lesquels simultanément -a¹=a²-a³=a⁴- est (a-4), -Z¹-Z²- est (b-2) et Y est (c-2) soient exclus.

2. Composé selon la revendication 1, **caractérisé en ce que** -a¹=a²-a³=a⁴- représente un radical bivalent de formule (a-3) ou (a-4).

3. Composé selon l'une quelconque des revendications 1 et 2, **caractérisé en ce que** -Z¹-Z²-représente un radical bivalent de formule (b-1), (b-2) ou (b-3), dans laquelle R⁷ représente un atome d'hydrogène ou un radical méthyle.

4. Composé selon l'une quelconque des revendications 1 à 3, **caractérisé en ce que** Y représente un radical bivalent de formule (c-1), dans laquelle n = 3, ou (c-2), dans laquelle m = 0 ou 1 et R⁸ représente un atome d'hydrogène.

5. Composé selon l'une quelconque des revendications 1 à 4, **caractérisé en ce que** X représente CR⁶ ; R², R³, R⁴ et R⁶ représentent chacun indépendamment un atome d'hydrogène, un radical halogéno, cyano, nitro ou hydroxy et R⁵ représente un atome d'hydrogène.

6. Composé selon l'une quelconque des revendications 1 à 5, **caractérisé en ce que** -a¹=a²-a³=a⁴-représente un radical bivalent de formule (a-3) ou (a-4); -Z¹-Z²- représente un radical bivalent de formule (b-1), (b-2) ou (b-3), dans laquelle R⁷ représente un atome d'hydrogène ou un radical méthyle; Y représente un radical bivalent de formule (c-1), dans laquelle n = 3, ou (c-2), dans laquelle m = 0 ou 1 et R⁸ représente un atome d'hydrogène; X représente CR⁶ ; R², R³, R⁴ et R⁶ représentent chacun indépendamment un atome d'hydrogène, un radical halogéno, cyano, nitro ou hydroxy et R⁵ représente un atome d'hydrogène.

7. Composé selon l'une quelconque des revendications 1 à 6 pour utilisation en tant que un médicament.

8. Composition pharmaceutique comprenant un vecteur ou un diluant pharmaceutiquement acceptable et, comme ingrédient actif, une quantité thérapeutiquement efficace d'un composé selon l'une quelconque des revendications 1 à 6.

9. Utilisation d'un composé selon l'une quelconque des revendications 1 à 6 pour la préparation d'un médicament pour la prévention et/ou le traitement d'un trouble ou d'une maladie qui répond à l'inhibition des récepteurs D₂, D₃ et/ou D₄ de la dopamine.

10. Utilisation d'un composé selon l'une quelconque des revendications 1 à 6 pour la préparation d'un médicament pour la prévention et/ou le traitement d'un trouble ou d'une maladie qui répond à l'inhibition de la recapture de la sérotonine et de l'antagoniste des récepteurs 5-HT_{1A}.

11. Utilisation d'un composé selon l'une quelconque des revendications 1 à 6 pour la préparation d'un médicament pour la prévention et/ou le traitement d'un trouble ou d'une maladie qui répond à l'effet combiné d'un antagoniste des récepteurs D₂, D₃ et/ou D₄ de la dopamine, d'un inhibiteur spécifique de la recapture de la sérotonine et d'un agoniste, d'un agoniste partiel ou d'un antagoniste des récepteurs 5-HT₁ₐ.

12. Utilisation d'un composé selon l'une quelconque des revendications 1 à 6 pour la préparation d'un médicament pour la prévention et/ou le traitement de troubles de l'humeur tels qu'un trouble anxieux général, un trouble panique, un trouble obsessionnel-compulsif, une dépression, une phobie sociale et un trouble du comportement alimentaire ; et d'autres troubles psychiatriques tels que, mais sans y être limités, la psychose et les troubles neurologiques.

13. Utilisation d'un composé selon l'une quelconque des revendications 1 à 6 pour la préparation d'un médicament pour la prévention et/ou le traitement de la schizophrénie.

14. Procédé de préparation d'un composé selon la formule (I), **caractérisé** soit par
(a) l'alkylation d'un intermédiaire de formule (III) avec un intermédiaire de formule (II), dans lesquelles toutes les variables sont telles que définies dans la revendication 1 et W représente un groupe partant approprié, dans un solvant inerte viv-à-vis de la réaction et éventuellement en présence d'une base appropriée ; 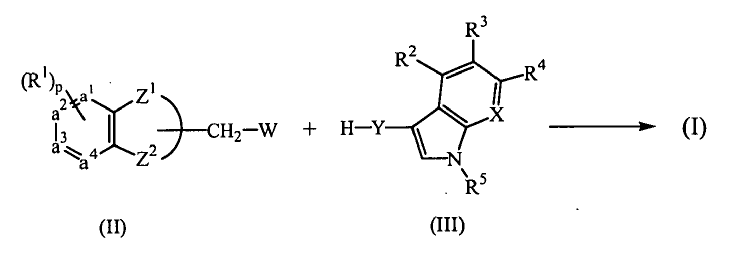
(b) l'amination réductrice d'un intermédiaire de formule (IV) avec un intermédiaire de formule (III) dans un solvant inerte viv-à-vis de la réaction en présence d'un agent réducteur. 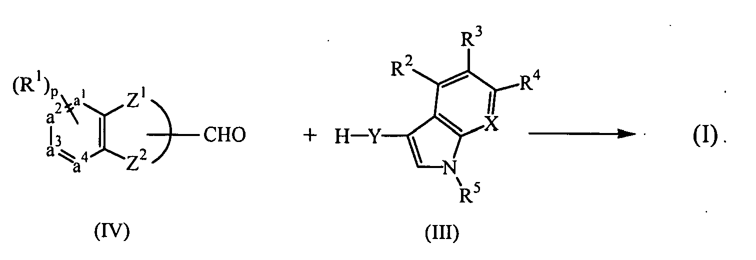
(c) la réaction d'un chlorure acide de formule (V) avec un intermédiaire de formule (III) dans un solvant inerte viv-à-vis de la réaction en présence d'une base appropriée, suivie par une réduction de l'intermédiaire amide correspondant formé dans un solvant inerte vis-à-vis de la réaction en présence d'un agent réducteur ; 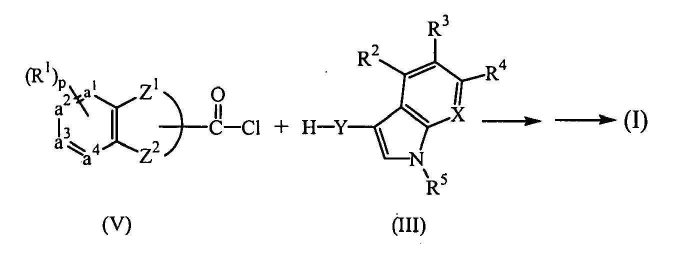
(d) et, si souhaitée, la transformation de composés de formule (I) l'un en l'autre suivant des transformations connues dans l'art et en outre, si souhaitée, la transformation des composés de formule (I) en un sel d'addition d'acide non toxique et thérapeutiquement actif par traitement avec un acide, ou en un sel d'addition de base non toxique et thérapeutiquement actif par traitement avec une base ou, inversement, la transformation de la forme du sel d'addition d'acide en une base libre par traitement avec un alcali, ou la transformation du sel d'addition de base en un acide libre par traitement avec un acide ; et, si souhaitée, la préparation de formes isomères stéréochimiques, de N-oxydes de ceux-ci et de sels d'ammonium quaternaire de ceux-ci.
